(19) Europäisches Patentamt
European Patent Office
Office européen des brevets

(11) EP 2 457 508 B1

(12) **EUROPÄISCHE PATENTSCHRIFT**

(45) Veröffentlichungstag und Bekanntmachung des Hinweises auf die Patenterteilung:
**21.05.2014 Patentblatt 2014/21**

(51) Int Cl.:
***A61B 5/05*** *(2006.01)* *A61B 5/145* *(2006.01)*

(21) Anmeldenummer: **10192473.6**

(22) Anmeldetag: **24.11.2010**

(54) **Erfassungsvorrichtung zum Erfassen eines Blutbildparameters**

Recording device for recording a blood count parameter

Dispositif de détection pour la détection d'un paramètre d'hémogramme

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**30.05.2012 Patentblatt 2012/22**

(73) Patentinhaber:
• **eesy-id GmbH**
**82166 Gräfelfing (DE)**
• **Friedrich-Alexander-Universität Erlangen-Nürnberg**
**91054 Erlangen (DE)**

(72) Erfinder: **Fischer, Georg**
**90425, Nürnberg (DE)**

(74) Vertreter: **Klinski, Robert et al**
**PATENTSHIP**
**Patentanwaltskanzlei**
**Elsenheimerstraße 65**
**80687 München (DE)**

(56) Entgegenhaltungen:
**WO-A1-2007/003955 WO-A1-2010/105373**
**US-A1- 2008 200 790 US-A1- 2010 069 731**
**US-B1- 6 332 087**

EP 2 457 508 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft das Gebiet der Erfassung einer Konzentration eines Blutbestandteiles, beispielsweise Zucker in durch ein Blutgefäß fließendes Blut.

Zur Bestimmung eines Blutbildparameters wie beispielsweise einer Konzentration eines Blutbestandteils kann invasiv Blut entnommen werden. Der Blutbildparameter kann dann unter Verwendung des entnommenen Bluts anhand standardisierter Teststreifen bestimmt werden, deren elektrische Widerstandswerte von der Konzentration des Blutbestandteiles, beispielsweise Blutzuckers, abhängen. Der jeweilige elektrische Widerstandswert kann beispielsweise unter Verwendung eines Blutzuckermessgerätes, das eine Gleichstrom-Widerstandsmessung zur Erfassung eines elektrischen Widerstandswertes eines Testreifens durchführt, erfasst werden. Der Widerstandswert kann anhand eines an sich bekannten Zusammenhanges zwischen einer Blutzuckerkonzentration und einem Widerstandswert in eine Blutzuckerkonzentration umgerechnet werden. Zur Erzielung einer hohen Erfassungsgenauigkeit ist jeder Teststreifen mit Kalibrierdaten, beispielsweise mit einem Referenzwiderstandswert oder mit einer entsprechenden Codierung, versehen, wodurch Variationen von Eigenschaften der Teststreifen ausgeglichen werden können. Nachteilig an invasiven Verfahren ist jedoch die Notwendigkeit der Blutentnahme und somit einer Verletzung eines Patienten. Außerdem ist eine kontinuierliche Erfassung einer Konzentration eines Blutbestandteils, um beispielsweise dessen Tagesgangkurve zu ermitteln, aufwändig. Darüber hinaus kann mittels des invasiven Verfahrens eine Zeitverzögerung zwischen einer Nahrungsaufnahme und beispielsweise einem Anstieg des Blutzuckers nicht genau erfasst werden. Ferner kann insbesondere bei einer geringen Konzentration des Blutzuckers in Blut ein Zeitpunkt der Insulinabgabe an den Patienten nicht genau bestimmt werden.

Zur nichtinvasiven Bestimmung eines Blutbildparameters wie beispielsweise einer Stoffkonzentration oder einer Stoffzusammensetzung in Blut können mikrowellenspektroskopische Verfahren eingesetzt werden. Die Mikrowellenspektroskopie zur Erfassung von Blutbildparametern basiert auf einer Einkopplung eines Mikrowellensignals in ein blutdurchströmtes Gewebe und einer Erfassung einer frequenzabhängigen Absorption eingekoppelter Mikrowellenenergie.

Die Druckschrift WO 20077003955 A1 betrifft ein Verfahren und ein Gerät zum Messen von einer Konzentration von Blutbestandteilen. Das Gerät umfasst eine Mikrowellenenergiequelle, um einen Bereich von Mikrowellenfrequenzen zu erzeugen. Ein Teil dieser Mikrowellenfrequenzen werden über eine erste Antenne in eine Gewebestruktur eingekoppelt. Eine zweite Antenne wird dazu verwendet, die von der ersten Antenne eingekoppelten Mikrowellen zu empfangen. Ein Datenprozessor ist dazu vorgesehen, die Konzentration von Blutbestandteilen anhand einer ermittelten Resonanzfrequenz zu bestimmen.

Die Druckschrift US 6,332,087 B1 beschreibt ein System und ein Verfahren zur nichtinvasiven bildgebenden Spektroskopie. Dazu wird eine Sender-Empfängerzusammenstellung verwendet, die eine Mehrzahl von Sender-Empfänger-Einheiten umfasst.

Die Druckschrift WO 20101105373 A1 betrifft ein Gerät zum Messen von zumindest einem Parameter, der von der dielektrischen Permittivität eines Gewebes abhängt.

Die Druckschrift US 201010069731 A1 betrifft ein System und Verfahren eines Verwendens einer nichtinvasive Echtzeit-Blutzuckermessung. Ein oder mehrere Sendeknoten sind mit einem Sender verbunden und werden zum Einkoppein von elektromagnetischer Strahlung verwendet, während ein oder mehrere Empfängerknoten mit einem Empfänger verbunden sind und die von den Sendeknoten ausgesendete Strahlung empfangen.

Die Druckschrift US 2008/0200790 A1 betrifft ein Gerät zum Messen von Blutzucker. Das Gerät umfasst zwei Sondenteile zum Senden und Empfangen von elektromagnetischer Strahlung.

In der Druckschrift von Andreas Caduff et al., "Non-invasive glucose monitoring in patients with Type 1 diabetes: A multi-sensor system combining sensors for dielectric and optical characterization of skin", Biosensors and Bioelectronics 24 (2009) 2778-2784, ist eine Mehrelektrodenanordnung zur mikrowellenbasierten Bestimmung eines Blutbildparameters beschrieben. Die Mehrelektrodenanordnung umfasst mehrere Elektrodenpaare mit unterschiedlichen Elektrodenabständen, durch welche unterschiedliche Eindringtiefen von Mikrowellensignalen realisiert werden können. Die Erfassung des Blutbildparameters erfolgt mittels einer Impedanzmessung, also mittels einer Eintormessung, und ist daher fehleranfällig bei etwaigen Impedanzfehlanpassungen. Aufgrund von unterschiedlichen Eindringtiefen ist mitunter keine Unterscheidung zwischen kapillarem und venösem Blut möglich, was die Messergebnisse verfälschen kann. Eine Messung eines Blutbildparameters an venösem Blut ist im Allgemeinen genauer als eine Messung des Blutbildparameters an kapillarem Blut, weil beispielsweise Blutzuckeränderungen in kapillarem Blut verzögert gegenüber venösem Blut sind.

In den Druckschriften von Buford Randal Jean et al., "A microwave frequency sensor for non-invasive blood-glucose measurement, SAS 2008 - IEEE Sensors Applications Symposium, Atlanta, GA, February 12 - 14, 2008, and M. McClung, Calibration methodology for a microwave non-invasive glucose sensor, Master Thesis, Baylor University, Mai 2008, ist eine weitere Elektrodenanordnung zur Bestimmung einer Blutzuckerkonzentration beschrieben. Dabei wird ausgenutzt, dass die dielektrischen Eigenschaften von Blut von einem Blutzuckergehalt abhängen. Durch Anpressen eines Daumens auf den Mikrowellensensor wird eine Änderung der Dielektrizitätszahl des Daumens durch eine Verstimmung eines Resonators gemessen. Durch das Anpressen des Daumes wird jedoch Blut verdrängt, was zu einer Verfälschung der Messergebnisse führen kann. Des Weiteren können die Messungen nicht kontinuierlich durchgeführt werden. Die Auswertung der Messdaten zur Bestimmung des Blutzuckergehalts hängt zudem von dem jeweiligen Patienten ab und ist daher bei anderen Patienten nicht reproduzierbar. Darüber hinaus ist mit diesem Verfahren die Eindringtiefe der Mikrowellenleistung nicht steuerbar, so dass eine Unterscheidung zwischen kapillarem und venösem Blut nicht möglich ist. Ferner wird die Änderung der Dielektrizitätszahl auf der Basis einer Eintormessung durchgeführt, welche anfällig bezüglich Fehlanpassungen ist.

Es ist daher die Aufgabe der vorliegenden Erfindung, ein genaueres Verfahren zum Erfassen eines Blutbildparameters, beispielsweise einer Konzentration von Blutzucker in Blut, zu schaffen.

Diese Aufgabe wird durch die Merkmale der unabhängigen Ansprüche gelöst. Vorteilhafte Weiterbildungsformen sind Gegenstand der abhängigen Ansprüche.

Die vorliegende Erfindung basiert auf der Erkenntnis, dass ein Blutbildparameter mikrowellenbasiert dann genau bestimmt werden kann, wenn die Mikrowellen direkt in das Blutgefäß eingekoppelt werden, so dass der Blutbildparameter anhand des venösen Bluts bestimmt werden kann.

Hierzu wird die weitere Erkenntnis ausgenutzt, dass ein Blutgefäß, das dieses umgebende Fettgewebe sowie eine Hautschicht als ein dielelektrisches Wellenleitersystem aufgefasst werden können, in dem sowohl transversal-elektrische, als auch transversal-magnetische Wellen ausbreitungsfähig sind. Um eine gezielte Mikrowelleneinkopplung in das Blutgefäß zu realisieren, können beispielsweise sendeseitig mehrere Sendeantennen und empfangsseitig mehrere Empfangsantennen vorgesehen sein. Durch eine Permutation aller Antennenkombinationen kann beispielsweise dasjenige Antennenpaar umfassend eine Sendeantenne um eine Empfangsantenne ausgewählt werden, das mit den geringsten Einkoppelverlusten verbunden ist. Das ausgewählte Antennenpaar kann dann zur mikrowellenbasierten Erfassung des Blutbildparameters herangezogen werden.

Gemäß einem Aspekt betrifft die Erfindung eine Erfassungsvorrichtung zum Erfassen eines Blutbildparameters von Blut in einem Blutgefäß, mit einem Sender mit einer Anzahl von Sendeantennen zum Aussenden von zumindest einem Sendesignal, einem Empfänger mit einer Anzahl von Empfangsantennen zum Empfangen von zumindest einem Empfangssignal, einem Prozessor, welcher ausgebildet ist, eine erste Erfassungskonfiguration umfassend eine Sendeantenne der Anzahl der Sendeantennen und eine Empfangsantenne der Anzahl der Empfangsantennen auszuwählen, und eine zweite Erfassungskonfiguration umfassend eine Sendeantenne der Anzahl der Sendeantennen und eine Empfangsantenne der Anzahl von Empfangsantennen auszuwählen, einem Verlustdetektor, welcher ausgebildet ist, bei Auswahl der ersten Erfassungskonfiguration zum Aussenden eines Sendesignals eine erste Verlustgröße auf der Basis des Sendesignals und eines Empfangssignals zu erfassen, und bei Auswahl der zweiten Erfassungskonfiguration zum Aussenden eines Sendesignals eine zweite Verlustgröße auf der Basis des Sendesignals und eines Empfangssignals zu erfassen, wobei der Prozessor ausgebildet ist, die Erfassungskonfiguration mit der geringeren Verlustgröße zum Erfassen des Blutbildparameters auszuwählen.

Bevorzugt werden die Sendesignale in Richtung des Blutgefäßes ausgesendet. Auf der Basis der Empfangssignale, welche empfangene Versionen der Sendesignale sind, sowie auf der Basis der Sendesignale kann beispielsweise dasjenige Antennenpärchen umfassend eine Sendeantenne und eine Empfangsantenne als diejenige Erfassungskonfiguration ausgewählt werden, die mit den geringsten Einkoppelverlusten verbunden ist. Die Einkoppelverluste können beispielsweise auf der Basis eines Vergleichs der vorgenannten Verlustgrößen, beispielsweise Absorptionslinien oder Dämpfungen, erfasst werden.

Gemäß einer Ausführungsform ist der Sender bei Auswahl der ersten Erfassungskonfiguration ausgebildet, das Sendesignal mittels der Sendeantenne der ersten Erfassungskonfiguration auszusenden, wobei der Empfänger bei Auswahl der ersten Erfassungskonfiguration ausgebildet ist, das Empfangssignal mittels der Empfangsantenne der ersten Erfassungskonfiguration zu empfangen, wobei der Sender bei Auswahl der zweiten Erfassungskonfiguration ausgebildet ist, das Sendesignal mittels der Sendeantenne der zweiten Erfassungskonfiguration auszusenden, und wobei der Empfänger bei Auswahl der zweiten Erfassungskonfiguration ausgebildet ist, das Empfangssignal mittels der Empfangsantenne der zweiten Erfassungskonfiguration zu empfangen, und wobei der Verlustdetektor ausgebildet ist, die erste Verlustgröße auf der Basis des Sendesignals und des Empfangssignals der ersten Erfassungskonfiguration zu erfassen, und die zweite Verlustgröße auf der Basis des Sendesignals und des Empfangssignals der zweiten Erfassungskonfiguration zu erfassen.

Gemäß einer Ausführungsform ist der Prozessor ausgebildet, die erste Verlustgröße mit der zweiten Verlustgröße zu vergleichen, um die geringere Verlustgröße der beiden Verlustgrößen zu bestimmen.

Gemäß einer Ausführungsform umfasst die Erfassungsvorrichtung eine Schaltmatrix, insbesondere eine durch den Prozessor steuerbare Schaltmatrix, welche ausgebildet ist, jeweils einen Ausgang einer Empfangsantenne der Anzahl der Empfangsantennen mit dem Verlustdetektor zu verbinden.

Gemäß einer Ausführungsform umfasst der Sender einen Sendesignalgenerator, wobei ein Ausgang des Sendesignalgenerators mittels einer Schaltmatrix, insbesondere mittels einer durch den Prozessor steuerbaren Schaltmatrix, mit jeweils einer Sendeantenne der Anzahl der Sendeantennen verbindbar ist.

Gemäß einer Ausführungsform ist der Sender ausgebildet, bei Auswahl der ersten Erfassungskonfiguration und bei Auswahl der zweiten Erfassungskonfiguration Sendesignale gleicher Frequenz, insbesondere einer Frequenz in einem Frequenzbereich zwischen 1 GHz und 15 GHz, oder gleicher Mode oder gleichen Wellentyps, insbesondere transversal-elektrisch oder transversal-magnetisch, auszusenden.

Gemäß einer Ausführungsform umfasst der Sender zumindest zwei Sendeantennen, wobei der Empfänger zumindest zwei Empfangsantennen umfasst. Die Sendeantennen und die Empfangsantennen können Dipolantennen oder Rahmenantennen oder Patch-Antennen sein.

Gemäß einer Ausführungsform umfasst der Verlustdetektor einen Netzwerkanalysator, insbesondere einen skalaren oder einen vektoriellen Netzwerkanalysator, oder einen Leistungsdetektor.

Gemäß einer Ausführungsform ist der Sender zum Erfassen des Blutbildparameters ausgebildet, unter Verwendung der Sendeantenne der ausgewählten Erfassungskonfiguration ein erstes Sendesignal mit einer ersten Frequenz und ein zweites Sendesignal mit einer zweiten Frequenz in das Blutgefäß einzukoppeln. Der Empfänger ist ausgebildet, unter Verwendung der Empfangsantenne der ausgewählten Erfassungskonfiguration ein erstes Empfangssignal bei der ersten Frequenz und ein zweites Empfangssignal bei der zweiten Frequenz zu empfangen. Der Verlustdetektor ist ausgebildet, eine erste Verlustgröße auf der Basis des ersten Sendesignals und des ersten Empfangssignals bei der ersten Frequenz zu bestimmen, und eine zweite Verlustgröße auf der Basis des zweiten Sendesignals und des zweiten Empfangssignals bei der zweiten Frequenz zu bestimmen. Der Prozessor ist ausgebildet, eine erste Frequenzverschiebung der ersten Verlustgröße relativ zu einer ersten Referenzverlustgröße zu bestimmen, eine zweite Frequenzverschiebung der zweiten Verlustgröße relativ zu einer zweiten Referenzverlustgröße zu ermitteln, und den Blutbildparameter auf der Basis der ersten Frequenzverschiebung und der zweiten Frequenzverschiebung zu ermitteln.

Gemäß einer Ausführungsform ist der Sender zum Erfassen des Blutbildparameters ausgebildet, unter Verwendung der Sendeantenne der ausgewählten Erfassungskonfiguration ein erstes Sendesignal mit einer ersten Frequenz und eines zweites Sendesignal mit einer zweiten Frequenz in das Blutgefäß einzukoppeln. Der Empfänger ist ausgebildet, unter Verwendung der Empfangsantenne der ausgewählten Erfassungskonfiguration ein erstes Empfangssignal bei der ersten Frequenz und ein zweites Empfangssignal bei der zweiten Frequenz zu empfangen. Der Prozessor ist ausgebildet, eine erste elektrische Verlustgröße auf der Basis des ersten Sendesignals und des ersten Empfangssignals zu ermitteln, eine zweite elektrische Verlustgröße auf der Basis des zweiten Sendesignals und des zweiten Empfangssignals zu ermitteln, eine Relaxationszeitkonstante eines Blutbestandteils in Abhängigkeit von der Frequenz mit der größeren elektrischen Verlustgröße zu bestimmen, und den Blutbildparameter auf der Basis eines vorbekannten Zusammenhanges zwischen der Relaxationszeitkonstanten und dem Blutbildparameter zu ermitteln.

Gemäß einer Ausführungsform ist der Verlustdetektor ausgebildet, zur Bestimmung der ersten Verlustgröße und der zweiten Verlustgröße eine Zweitormessung durchzuführen, insbesondere mittels der Zweitormessung jeweils einen Vorwärtstransmissionsfaktor $S_{21}$ zu bestimmen.

Gemäß einer Ausführungsform ist der Verlustdetektor ausgebildet, die erste Verlustgröße und die zweiten Verlustgröße jeweils auf der Basis der folgenden Formel zu bestimmen:

$$P_{Verlust} = 1 - \left| S_{11} \right|^2 - \left| S_{21} \right|^2,$$

wobei $P_{Verlust}$ die jeweilige Verlustgröße bezeichnet, und wobei $S_{11}$ den Eingangsreflexionsfaktor und $S_{21}$ den Vorwärtstransmissionsfaktor bezeichnen.

Gemäß einer Ausführungsform ist der Sender ausgebildet, das Sendesignal als eine Mode oder als einen Wellentyp, insbesondere eine transversal-elektrische (TE) Welle oder als eine transversal-magnetische (TM) Welle oder eine transversal-elektromagnetische (TEM) Welle oder eine HE-Welle einzukoppeln, insbesondere longitudinal oder transversal bezüglich eines Verlaufes des Blutgefäßes oder der Richtung des Blutflusses einzukoppeln.

Gemäß einer Ausführungsform ist der Blutbildparameter eine Konzentration eines Blutbestandteils, insbesondere Zuckers wie Glucose, oder Laktats oder Milchsäure oder Sauerstoffs.

Gemäß einem weiteren Aspekt betrifft die Erfindung ein Verfahren zum Erfassen eines Blutbildparameters von Blut in einem Blutgefäß unter Verwendung eines Senders mit einer Anzahl von Sendeantennen zum Aussenden von zumindest einem Sendesignal und eines Empfängers mit einer Anzahl von Empfangsantennen zum Empfangen von zumindest einem Empfangssignal, mit Auswählen einer ersten Erfassungskonfiguration umfassend eine Sendeantenne der Anzahl der Sendeantennen und eine Empfangsantenne der Anzahl von Empfangsantennen auszuwählen, bei Auswahl der ersten Erfassungskonfiguration zum Aussenden eines Sendesignals, Erfassen einer ersten Verlustgröße auf der Basis des Sendesignals und eines Empfangssignals, Auswählen einer zweiten Erfassungskonfiguration umfassend eine Sendeantenne der Anzahl der Sendeantennen und eine Empfangsantenne der Anzahl von Empfangsantennen, bei Auswahl der zweiten Erfassungskonfiguration zum Aussenden eines Sendesignals, Erfassen einer zweite Verlustgröße auf der Basis des Sendesignals und eines Empfangssignals, und Auswählen die Erfassungskonfiguration mit der geringeren Verlustgröße zum Erfassen des Blutbildparameters.

Weitere Verfahrensschritte ergeben sich unmittelbar aus der Funktionalität der Erfassungsvorrichtung.

Weitere Ausführungsbeispiele werden Bezug nehmend auf die beiliegenden Figuren erläutert. Es zeigen:

Fig. 1 ein Blockdiagramm einer Erfassungsvorrichtung;

Fig. 2 ein Blockdiagramm einer Erfassungsvorrichtung;

Fig. 3 ein Modell eines Querschnitts eines menschlichen Unterarms;

Fig. 4A bis 4D Antennen;

Fig. 5 eine elektrische Dipolantenne;

Fig. 5B eine Erregeranordnung;

Fig. 6A, 6B Erregeranordnungen;

Fig. 7A eine Schleifenantenne 601;

Fig. 7B eine Erregeranordnung;

Fig. 8 eine Erregeranordnung;

Fig. 9 eine Erregeranordnung;

Fig. 10 eine Erregeranordnung;

Fig. 11 eine Erregeranordnung;

Fig. 12 ein prinzipielles Schaltbild einer Erfassungsvorrichtung;

Fig. 13 eine Frequenzverschiebung eines Absorptionsmaximums;

Fig. 14 Transmissionsverhalten;

Fig. 15 Frequenzverschiebungen;

Fig. 16 ein Diagramm zur Illustrierung der reellen Dielektrizitätszahl $\varepsilon'$ und der komplexen Dielektrizitätszahl $\varepsilon''$ in Abhängigkeit der Frequenz;

Fig. 17 ein Diagramm zur Illustrierung eines Zusammenhangs zwischen der Relaxationszeitkonstanten ($\tau$) und der Glucosekonzentration im Blut;

Fig. 18 ein schematisches Blockdiagramm einer Erfassungsvorrichtung mit einer Kommunikationsvorrichtung;

Fig. 19 ein schematisches Blockdiagramm eines Armbandes;

Fig. 20 ein schematisches Blockdiagramm eines Ausschnittes eines Armbandes;

Fig. 21 ein schematisches Blockdiagramm eines Ausschnittes Armbandes; und

Fig. 22 ein schematisches Blockdiagramm einer Anordnung der Elektroden der Erfassungsvorrichtung.

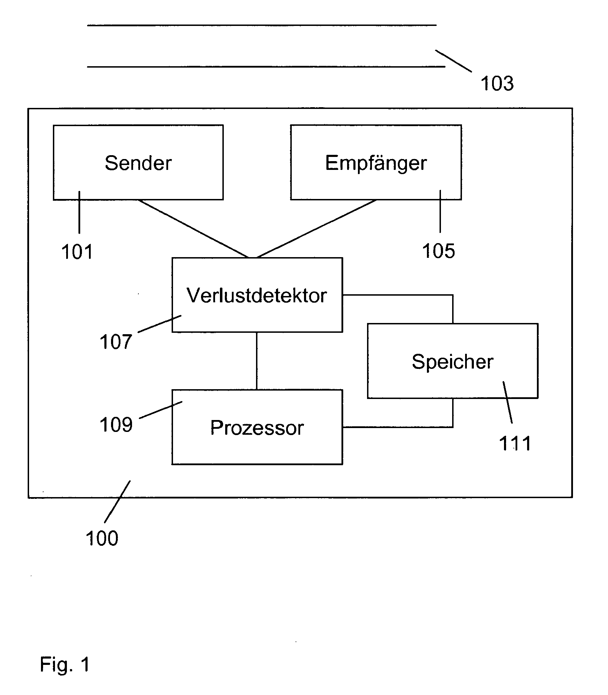

Fig. 1 zeigt ein Blockdiagramm einer Erfassungsvorrichtung 100 zum Erfassen eines Blutbildparameters wie beispielsweise einer Konzentration von Blutzucker. Die Erfassungsvorrichtung umfasst einen Sender 101, welcher ausgebildet ist, in das in Fig. 1 schematisch dargestellte Blutgefäß 103 zumindest ein Sendesignal, beispielsweise ein Mikrowellensignal, einzukoppeln.

Die Erfassungsvorrichtung 100 umfasst ferner einen Empfänger 105, welcher ausgebildet ist, zumindest ein Empfangssignal zu empfangen.

Die Erfassungsvorrichtung 100 umfasst ferner einen Verlustdetektor 107, welcher beispielsweise einen Leistungsdetektor umfassen kann. Der Verlustdetektor 107 ist mit dem Sender 101 und mit dem Empfänger 105 gekoppelt.

Die Erfassungsvorrichtung umfasst ferner einen Prozessor 109, welcher mit dem Verlustdetektor 107 gekoppelt ist.

Die Erfassungsvorrichtung 100 kann optional einen Speicher 111 aufweisen, auf welchen beispielsweise der Prozessor 109 und optional der Verlustdetektor 107 zugreifen können.

Der Sender 101 kann beispielsweise eine oder mehrere Sendeantennen zum Aussenden von einem oder mehreren Sendesignalen aufweisen, welche beispielsweise als Dipolantennen oder Rahmenantennen oder Patch-Antennen ausgebildet sein können. In Analogie hierzu kann der Empfänger 105 eine oder mehrere Sendeantennen zum Empfangen von einem oder mehreren Empfangssignalen aufweisen. Der Prozessor 109 ist bevorzugt ausgebildet, eine Mehrzahl von Erfassungskonfigurationen nacheinander auszuwählen. Dabei umfasst jede Erfassungskonfiguration eine einzige Sendeantenne und eine einzige Empfangsantenne, wobei die Sendeantennen voneinander beabstandet und wobei die Empfangsantennen voneinander beabstandet sein können. Bei Auswahl einer Erfassungskonfiguration erregt der Sender 101 die dazu gehörige Sendeantenne zum Aussenden eines Sendesignals, wobei der Empfänger 105 die jeweilige Empfangsantenne verwendet, um ein Empfangssignal zu empfangen.

Auf der Basis des Sendesignals und des Empfangssignals kann der Verlustdetektor 107 beispielsweise eine elektromagnetische Verlustgröße wie Energieabsorption bestimmen. Im nächsten Schritt wird eine weitere Erfassungskonfiguration zum Aussenden eines Sendesignals verwendet und es wird eine weitere Verlustgröße erfasst. Auf diese Weise werden mehrere Erfassungskonfigurationen der Reihe nach oder in beliebiger Reihenfolge zum Einkoppeln eines Sendesignals in das Blutgefäß 103 verwendet, wobei bei jeder Erfassungskonfiguration eine Verlustgröße mittels des Verlustdetektors 107 bestimmt wird. Der Prozessor 109 kann die Verlustgrößen beispielsweise vergleichen und diejenige Erfassungskonfiguration auswählen, welche mit der geringsten Verlustgröße verbunden ist. Die ausgewählte Erfassungskonfiguration wird zum Erfassen des Blutbildparameters, wie es nachfolgend beschrieben ist, verwendet.

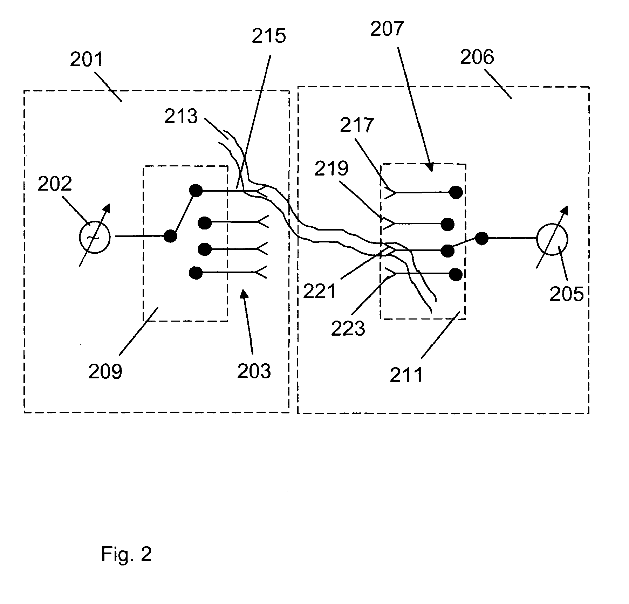

Fig. 2 zeigt eine Erfassungsvorrichtung, welche eine Ausführungsform der in Fig. 1 dargestellten Erfassungsvorrichtung 100 sein kann. Die Erfassungsvorrichtung umfasst einen Sender 201, der beispielsweise einen durchstimmbaren Oszillator 202 aufweisen kann, sowie eine Mehrzahl von Sendeantennen 203. Die Erfassungsvorrichtung umfasst ferner einen Verlustdetektor 205, welcher beispielsweise einen Leistungsdetektor aufweisen kann. Ferner ist ein Empfänger 206 mit einer Mehrzahl von Empfangsantennen 207 vorgesehen.

Ein Ausgang des durchstimmbaren Oszillators 202 ist beispielsweise mittels einer Schaltmatrix 209 mit jedem Antenneneingang beispielsweise der Reihe nach oder in beliebiger Reihenfolge schaltbar verbindbar. In Analogie hierzu ist jeder Ausgang einer Empfangsantenne der Mehrzahl der Empfangsantennen 207 mittels einer Schaltmatrix 211 mit dem Verlustdetektor 205 verbindbar.

Die Schaltmatrizen 209, 211 können Schalter, insbesondere Transistorschalter, aufweisen.

Mittels der Schaltmatrix 211 sowie der Schaltmatrix 209 kann beispielsweise dasjenige Paar umfassend eine Sendeantenne und eine Empfangsantenne ausgewählt werden, das eine optimale Einkopplung eines Mikrowellensignals in ein in Fig. 2 schematisch dargestelltes Blutgefäß 213 ermöglicht. Mittels der Schaltmatrizen 209 und 211 werden die Antennenpaare der Reihe nach ausgewählt beginnend beispielsweise mit einer ersten Sendeantenne 215, mittels welcher ein Sendesignal ausgesendet wird.

Empfangsseitig werden mittels der Schaltmatrix 211 der Reihe nach die Empfangsantennen beginnend beispielsweise mit der Empfangsantenne 217 zum Empfang eines entsprechenden Empfangssignals ausgewählt, wobei auf der Basis des Sendesignals und des Empfangssignals eine Verlustgröße erfasst wird. Im nächsten Schritt wird beispielsweise die Empfangsantenne 219 ausgewählt, wobei wiederum auf der Basis des Sendesignals und eines durch die Empfangsantenne 219 empfangenen Empfangssignals eine Verlustgröße mittels des Verlustdetektors erfasst wird. Danach wird beispielsweise die Empfangsantenne 221 ausgewählt, wobei auf der Basis des Sendesignals und eines Empfangssignals eine weitere Verlustgröße erfasst wird. Im nächsten Schritt wird die Empfangsantenne 223 ausgewählt und es wird auf der Basis des Sendesignals sowie eines durch die Empfangsantenne 223 empfangenen Empfangssignals eine weitere Verlustgröße bestimmt. Im nächsten Schritt kann die Schaltmatrix 209 beispielsweise eine weitere Sendeantenne auswählen, wobei die vorgenannten Schritte wiederholt werden können. Durch einen Vergleich der ermittelten Verlustgrößen wird beispielsweise die kleinste Verlustgröße ermittelt. In dem in Fig. 2 dargestellten Beispiel ist beispielsweise zu erwarten, dass die Erfassungskonfiguration mit der Sendeantenne 215 und der Empfangsantenne 221 mit den geringsten Einkoppelverlusten behaftet ist, weil die Antennen 215, 221 unmittelbar oberhalb des Blutgefäßes liegen und somit eine Einkopplung eines Signals in das Blutgefäß 213 ermöglichen. Die ausgewählte Erfassungskonfiguration kann beispielsweise zur Erfassung eines Blutbildparameters herangezogen werden. Die vorstehend beschriebenen Auswahlschritte können in beliebiger Reihenfolge durchgeführt werden. So können beispielsweise für die Sendeantenne 215 alle oder einige der Empfangsantennen 207 getestet werden.

Die Sendeantennen 203 bzw. die Empfangsantennen 207 können sich hinsichtlich ihres Ortes und/oder hinsichtlich ihrer Feldkomponente, welche dominant angeregt werden soll, unterscheiden. Durch die Schaltmatrizen 209 und 211 ist dabei gewährleistet, dass für die jeweils gewählte Frequenz der optimale Erregertyp, beispielsweise Schleifenanenne, elektrische Dipolantenne, Patch-Antenne oder Erregerort, ausgewählt werden kann.

Die in Fig. 2 dargestellte Erfassungsvorrichtung kann beispielsweise in einem aufpumpbaren Armband integriert sein. Zwischen den Erfassungen der Verlustgrößen, welche beispielsweise durch Messungen der Steuerparameter erfolgen können, kann Luft aus dem Armband abgelassen werden, so dass die Haut belüftet wird und sich kein Schweiß bildet. Ein Zeitabstand zwischen den Messungen kann dabei variabel erfolgen. Die Messungen können beispielsweise in Abständen von 10 Minuten durchgeführt werden. Je nach Bedarf können jedoch häufigere Messungen durchgeführt werden, wobei die Häufigkeit der Messungen beispielsweise durch die Zeitpunkte der Einnahme der Mahlzeiten bestimmt sein kann.

Da insbesondere in den Pausen zwischen den Messungen die auf der Haut liegenden Sende- bzw. Empfangsantennen, welche jeweils durch eine Elektrodenplatte gebildet sein können, verrutschen können, kann durch die in Fig. 2 dargestellte Auswahl mehrerer Erreger sichergestellt werden, dass ein Erreger ausgewählt wird, welcher über dem Blutgefäß 213 liegt. Mittels der jeweiligen Umschaltmatrix 209 und 211 kann somit derjenige Erreger ausgewählt werden, welcher ein Maximum einer Einkopplung von Mikrowellenenergie in das Blutgefäß 213 ermöglicht.

Im folgenden wird die Bestimmung des Blutbildparameters beispielhaft anhand der in Fig. 1 dargestellten Erfassungsvorrichtung 100 beschrieben. Die nachfolgenden Ausführungen gelten jedoch analog auch für die in Fig. 2 dargestellte Erfassungsvorrichtung.

Zum Bestimmen des Blutbildparameters kann der Sender 101 gemäß einer Ausführungsform beispielsweise das in Fig. 1 schematisch dargestellte Blutgefäß 103 ein erstes Sendesignal mit einer ersten Frequenz und ein zweites Sendesignal mit einer zweiten Frequenz einzukoppeln. Das erste Sendesignal und das zweite Sendesignal können beispielsweise gemeinsam ein Breitbandsignal ergeben. Der Sender 101 kann ausgebildet sein, das erste Sendesignal und das zweite Sendesignal hintereinander, beispielsweise durch einen Frequenz-Sweep, auszusenden. Hierzu kann der Sender 101 eine oder mehrere Sendeantennen, welche beispielsweise als Dipolantennen oder Rahmenantennen oder Patch-Antennen ausgebildet sein können, aufweisen.

Die Erfassungsvorrichtung 100 umfasst ferner den Empfänger 105, welcher ausgebildet sein kann, ein erstes Empfangssignal bei der ersten Frequenz und ein zweites Empfangssignal bei der zweiten Frequenz zu empfangen. Hierzu kann der Empfänger eine oder mehrere Empfangsantennen aufweisen.

Die Erfassungsvorrichtung 100 umfasst ferner den Verlustdetektor 107, welcher beispielsweise mit dem Sender 101 und dem Empfänger 105 gekoppelt und vorgesehen sein kann, eine erste Verlustgröße auf der Basis des ersten Sendesignals und des ersten Empfangssignals sowie eine zweite Verlustgröße auf der Basis des zweiten Sendesignals und des zweiten Empfangssignals zu bestimmen.

Die Erfassungsvorrichtung 100 umfasst ferner den Prozessor 109, welcher mit dem Verlustdetektor 107 gekoppelt und vorgesehen sein kann, eine erste Frequenzverschiebung der ersten Verlustgröße relativ zu einer ersten Referenzverlustgröße und eine zweite Frequenzverschiebung der zweiten Verlustgröße relativ zu einer zweiten Referenzverlustgröße zu bestimmen. Der Prozessor 109 kann ferner ausgebildet sein, den Blutbildparameter auf der Basis der beiden Frequenzverschiebungen zu bestimmen.

Die Erfassungsvorrichtung 100 kann ferner den Speicher 111 aufweisen, auf welchen beispielsweise der Prozessor 109 und optional der Verlustdetektor 107 zugreifen können. In dem Speicher 111 sind beispielsweise die erste und die zweite Referenzverlustgröße oder eine Mehrzahl von Referenzverlustgrößen abgelegt. Bei den Referenzverlustgrößen kann es sich beispielsweise um Absorptionen oder Absorptionslinien einer Wasserlösung mit einem Blutbestandteil, beispielsweise Blutzucker, handeln. Die auf der Basis der Frequenzverschiebungen erfassten Verlustgrößen können frequenzverschobene Absorptionen oder Absorptionslinien sein, so dass auf der Basis der Frequenzverschiebungen der Blutbildparameter, wie beispielsweise eine Konzentration von Blutzucker, ermittelt werden kann.

Die in Fig. 1 oder in Fig. 2 dargestellte Erfassungsvorrichtung 100 nutzt die Erkenntnis aus, dass ein Blutgefäß, eine Hautschicht sowie ein das Blutgefäß umgebendes Fettgewebe von beispielsweise einem menschlichen Unterarms als ein dielektrisches Wellenleitersystem aufgefasst werden können. Der Aufbau eines menschlichen Unterarms ist beispielsweise in Netter, F. N., Atlas der Anatomie, Thieme Verlag, 2006, beschrieben. Ein menschlicher Unterarm besteht aus zwei Knochen, welche von einem Muskelgewebe umgeben sind. Um das Muskelgewebe herum sind oberflächliche Venen, d.h. Blutgefäße, verteilt. Die Knochen, das Muskelgewebe sowie die Venen sind durch ein Fettgewebe ummantelt, welches durch obere Hautschichten bedeckt ist. Die oberflächlichen Venen sind relativ nahe an den oberen Hautschichten angeordnet und von diesen durch das Fettgewebe separiert. Werden beispielsweise der in Fig. 1 dargestellte Sender 101 und der Empfänger 105 auf die obere Hautschicht aufgelegt, so kann der Sender 101 dazu eingesetzt werden, in das durch ein Blutgefäß, ein Fettgewebe sowie eine Hautschicht gebildete dielektrische Wellenleitersystem beispielsweise eine transversal-elektrische (TE) Welle oder eine transversal-magnetische (TM) Welle einzukoppeln. Dabei kann die Hautschicht sowie das Fettgewebe als ein Filmwellenleiter verstanden werden.

Wird beispielsweise ein Mikrowellenmesskopf, wie er für die Bestimmung einer komplexen Dielektrizitätszahl von Materialien benutzt werden kann, verwendet, so kann dadurch das Stoffgemisch bestehend aus Haut, Fettgewebe und Venen charakterisiert werden.

Um einen Blutbildparameter zu erfassen, ist es vorteilhaft, im Wesentlichen nur das venöse Blut zu erfassen. Hierzu kann der Sender 101 ausgebildet sein, das Sendesignal in der Gestalt einer elektromagnetischen Welle direkt in das Blutgefäß 103 einzukoppeln. Der Sender 101 sowie der Empfänger 105 können jeweils eine Mehrzahl von Antennen aufweisen, so dass zur Einkopplung der elektromagnetischen Welle in das Blutgefäß und zur Auskopplung einer elektromagnetischen Welle aus dem Blutgefäß 103 jeweils diejenige Sendeantenne und diejenige Empfangsantenne ausgewählt werden können, welche mit den geringsten Koppelverlusten verbunden sind.

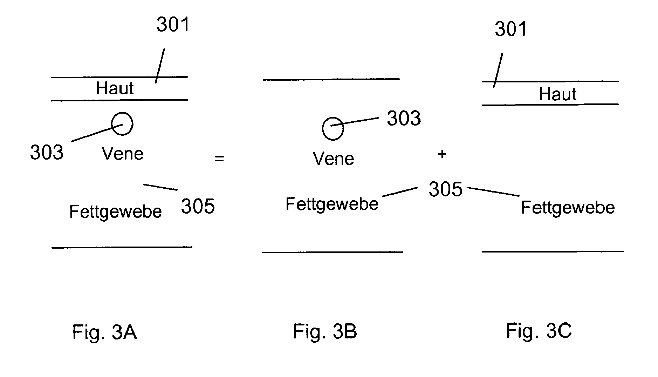

In den Figuren 3A bis 3C ist ein vereinfachtes Modell eines Querschnitts eines menschlichen Unterarms, z.B. eines Handgelenks, wie es beispielsweise für Feldsimulationen bzw. zur Modellierung eines dielektrischen Wellenleitersystems benutzt werden kann. Wie in Fig. 3A dargestellt, umfasst das Modell eine Hautschicht 301, ein Blutgefäß 303 sowie ein das Blutgefäß 303, z.B. eine Vene, umgebendes Fettgewebe 305. Das in Fig. 3A dargestellte Modell bildet ein dielektrisches Wellenleitersystem umfassend den in Fig. 3B dargestellten dielektrischen Wellenleiter sowie den in Fig. 3C dargestellten elektrischen Filmwellenleiter.

Der in Fig. 3B dargestellte dielektrische Wellenleiter umfasst das Blutgefäß 303 sowie das dieses umgebende Fettgewebe 305. Der dielektrische Filmwellenleiter aus Fig. 3C umfasst hingegen die Hautschicht 301 sowie das Fettgewebe 305. Für die Hautschicht 301, für das Fettgewebe 305 sowie für das Blutgefäß 303 kann jeweils ein unterschiedliches dispersives, d.h. frequenzabhängiges, Verhalten der jeweiligen komplexen Dielektrizitätszahl angesetzt werden. Das oben liegende Blutgefäß 303 wird dabei als ein dielektrischer Wellenleiter interpretiert, in dem je nach Frequenz verschiedene Moden bzw. Wellentypen, beispielsweise eine TE-Welle, eine TM-Welle, eine TEM-Welle oder eine HE-Welle, ausbreitungsfähig sein können. Zu dem Wellenleitermechanismus in dem dielektrischen Wellenleiter kommt ein weiterer Wellenleitermechanismus in der Gestalt des in Fig. 3C dargestellten Filmwellenleiters hinzu, der durch die obere Hautschicht 301 gebildet wird.

Eine Sendeantenne des Senders 101 sowie eine Empfangsantenne des Empfängers 105 können bevorzugt derart ausgestaltet sein, dass sie dezidiert Mikrowellenleistung in das Blutgefäß 303 einkoppeln und diese beispielsweise nach einigen Zentimetern wieder auskoppeln. Das Blutgefäß 303 dient dabei als eine Messstrecke und ist somit als ein verteiltes Element und nicht mehr als ein konzentriertes Element zu sehen. Bevorzugt wird die Messung der Verlustgrößen auf der Basis einer Zweitormessung durchgeführt. Dabei können insbesondere bei einer Ankopplung der Erfassungsvorrichtung an ein Handgelenk Primärmoden in dem dielektrischen Wellenleiter 3B angeregt werden, so dass eine Anregung von Filmwellenleitermoden in dem Filmwellenleiter 3C vermieden wird, wodurch die Erfassung des Blutbildparameters genauer durchgeführt werden kann.

Um Primärmoden im dielektrischen Wellenleitersystem anzuregen, kann berücksichtigt werden, dass je nach gewählter Frequenz eines Sendesignals unterschiedliche Moden dominant sein können. Bevorzugt sind Modentypen, die eine Konzentration der Felder in dem Blutgefäß 303 aufweisen, solchen Moden vorzuziehen, bei denen die Felder in der Hautschicht 301 konzentriert sind. Aufgrund der dielektrischen Eigenschaften des in Fig. 3B dargestellten dielektrischen Wellenleiters zeigt sich, dass für bestimmte Modentypen longitudinale Komponenten $E_{longitudinal}$, $H_{longitudinal}$ in Ausbreitungsrichtung, d.h. in Richtung eines Blutgefäßverlaufs, stärker als die transversalen Komponenten $E_{transversal}$, $H_{transversal}$, d.h. quer zum Blutgefäßverlauf, sind. Bevorzugt werden daher in dem zu erfassenden Frequenzbereich diejenigen Moden angeregt, welche eine maximale Einkopplung der Mikrowellenleistung in das Blutgefäß 303 ermöglichen.

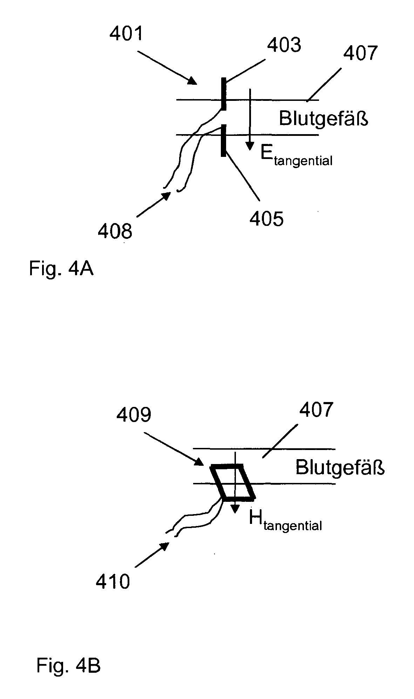

In den Figuren 4A bis 4D sind beispielhaft einige Antennen dargestellt, welche als Sendeantennen, d.h. Erreger, oder auch als Empfangsantennen eingesetzt werden können.

Die in Fig. 4A dargestellte Antenne 401 ist als ein elektrischer Dipol mit einem ersten Antennenabschnitt 403 und einem zweiten Antennenabschnitt 405 ausgeführt Die Antennenabschnitte 403 und 405 sind voneinander beabstandet und beispielsweise quer zu einem Verlauf eines Blutgefäßes 407 angeordnet. Eine Anregung der Antenne 401 kann durch Zuleitungen 408 erfolgen. Ein derart angeordneter elektrischer Dipol kann beispielsweise ein elektrisches Feld $E_{tangential}$ erzeugen, das quer zum Verlauf des Blutgefäßes bzw. zur Blutflussrichtung zeigt.

In Fig. 4B ist eine Antenne 409 dargestellt, welche eine Rahmenantenne sein kann. Die Rahmenantenne kann beispielsweise eine Viereckform oder eine runde Form aufweisen. Bei der in Fig. 4B dargestellten Anordnung der Rahmenantenne 409 bezüglich des Blutgefäßes 407 wird beispielsweise ein magnetisches Feld $H_{tangential}$ angeregt, das quer zum Verlauf des Blutgefäßes 407 bzw. quer zur Blutflussrichtung zeigt. Eine Anregung der Antenne 409 kann durch Zuleitungen 410 erfolgen.

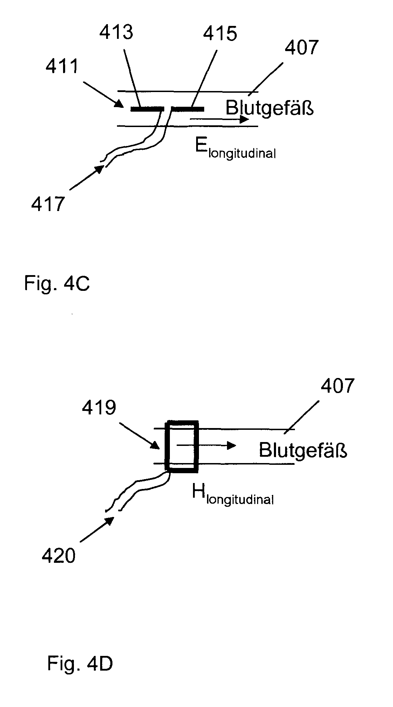

In Fig. 4C ist eine Antenne 411 dargestellt, welche einen elektrischen Dipol mit einem ersten Antennenabschnitt 413 und einem zweiten Antennenabschnitt 415 bildet. Die Antennenabschnitte 413 und 415 sind voneinander beabstandet und werden mittels der in Fig. 4C dargestellten Zuleitungen 417 angeregt. Der durch die Antenne 411 gebildete elektrische Dipol ist derart bezüglich des Verlaufs des Blutgefäßes 407 angeordnet, dass die Abschnitte 413 und 415 parallel zum Verlauf des Blutgefäßes 407 angeordnet sind. Dadurch wird ein in Richtung des Verlaufs des Blutgefäßes weisendes elektrisches Feld mit der Feldkomponente $E_{longitudinal}$ angeregt.

Fig. 4D zeigt eine Rahmenantenne 419, welche beispielsweise in der Gestalt eines viereckigen oder runden Rahmens, welcher eine Schleifenantenne bildet, gebildet sein kann. Die Rahmenantenne 419 wird mittels Zuleitungen 320 angeregt und ist wie es in Fig. 4D dargestellt ist, bezüglich des Verlaufs des Blutgefäßes 407 bzw. bezüglich der Blutflussrichtung derart angeordnet, dass das magnetische Feld eine in Richtung des Verlaufs des Blutgefäßes 407 weisende Komponente $H_{longitudinal}$ aufweist.

Der jeweils zu messende Frequenzbereich richtet sich beispielsweise danach, welche Spektrallinien, d.h. welche Absorptionslinien, zu erfassen sind. Es können beispielsweise die charakteristischen Absorptionslinien eines Stoffes oder auch ein Effekt beobachtet werden, den ein bestimmter Blutbestandteil auf die Absorptionslinien von Wasser bzw. von einer Wasserlösung mit einer Konzentration des Blutbestandteils aufweist.

Bei den in den Figuren 4A bis 4D dargestellten Antennen handelt es sich entweder um elektrische Dipole oder um magnetische Rahmenantennen. Darüber hinaus können auch Patch-Antennen eingesetzt werden. Elektrische Dipole erzeugen dominant ein elektrisches Feld in der Achse des elektrischen Dipols. Diese Achse kann entweder, wie es in Fig. 4A dargestellt ist, tangential zum Blutgefäß 407 bzw. zur Blutflussrichtung, oder wie es in Fig. 4C dargestellt ist, in Richtung des Blutgefäßes 407 bzw. in Blutflussrichtung ausgerichtet sein. Falls primär ein magnetisches Feld erzeugt werden soll, so kann eine Rahmenantenne als Erreger eingesetzt werden. Ist ein Flächenvektor auf der von dem die Rahmenantenne bildenden Rahmen aufgespannten Fläche quer zu dem Blutgefäß 407 bzw. quer zur Blutflussrichtung ausgerichtet, so ist auch das magnetische Feld quer zu dem Blutgefäß 407 ausgerichtet, wie es in Fig. 4B dargestellt ist. Zeigt der Flächenvektor hingegen in Richtung des Blutgefäßes 407, so ist auch das Magnetfeld in Richtung des Blutgefäßes 407 ausgerichtet, wie es beispielsweise in Fig. 4B dargestellt ist. Aus der Wahl eines der in den Figuren 4A bis 4D dargestellten Erregers ergibt sich dann beispielsweise die dominant angeregte Mode bzw. Wellentyp.

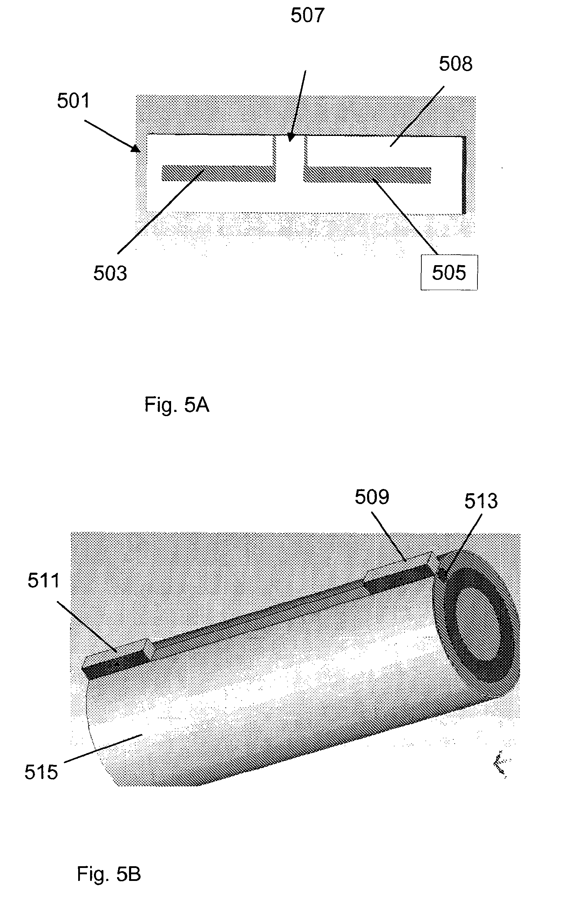

Fig. 5A zeigt eine elektrische Dipolantenne 501, welche als eine Sendeantenne oder als eine Empfangsantenne eingesetzt werden kann. Die elektrische Dipolantenne 501 umfasst Dipolantennenabschnitte 503 und 505, welche in oder auf einem Substrat 508 angeordnet sind und mittels Zuleitungen 507 angeregt werden können. Die Dipolantenne 501 kann als Sendeantenne oder als Empfangsantenne eingesetzt werden.

Fig. 5B zeigt eine Erregeranordnung einer Sendeantenne 509 eines Senders und einer Empfangsantenne 511 eines Empfängers in Richtung eines Verlaufs eines Blutgefässes 513 unterhalb einer Hautschicht 515. Die Sendeantenne 509 und die Empfangsantenne 511 sind beispielsweise elektrische Dipolantennen gemäß Fig. 5A. Bei der in Fig. 5B dargestellten Anordnung wird ein elektrisches Feld mit einer Feldkomponente in Richtung des Verlaufs des Blutgefässes 513, bzw. in Blutflussrichtung erzeugt.

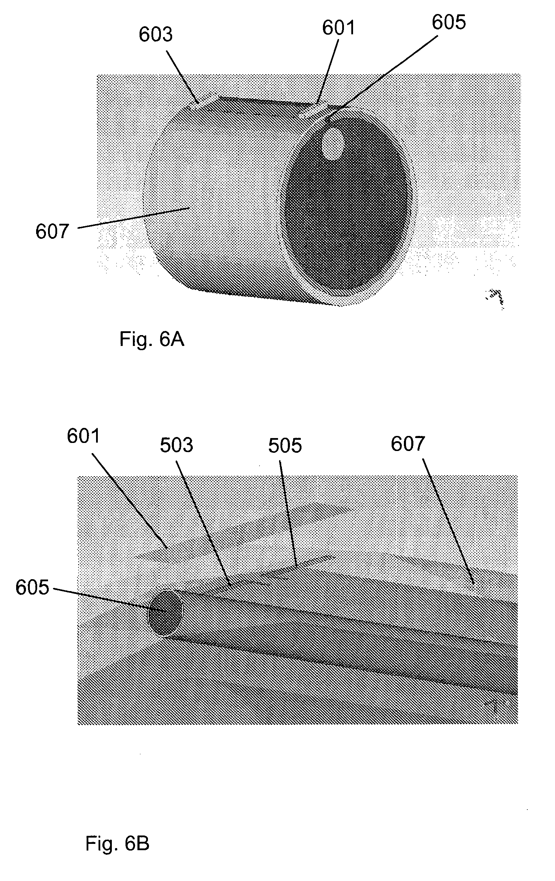

Fig. 6A zeigt eine Erregeranordnung umfassend eine Sendeantenne 601 eines Senders und eine Empfangsantenne 603 eines Empfängers quer zur Ausbreitungsrichtung eines Blutgefäßes 605, d.h. quer zur Blutflussrichtung, das unter einer Hautschicht 607 liegt. Die Sendeantenne 601 und die Empfangsantenne 603 können jeweils beispielsweise durch die in Fig. 5A dargestellte elektrische Dipolantenne gebildet sein. In Fig. 6B ist die

Anordnung der Dipolantennenabschnitte 503 und 505 bezüglich der Blutflussrichtung detaillierter dargestellt.

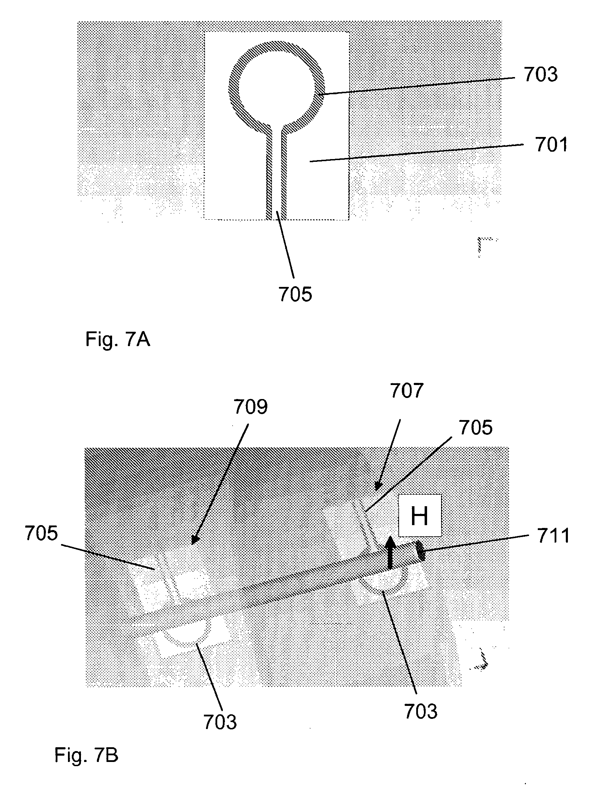

Fig. 7A zeigt eine Schleifenantenne 701 mit einem kreisförmigen Rahmen 703 und Zuleitungen 705 zum Anregen des kreisförmigen Rahmens 703. Die Schleifenantenne 701 kann beispielsweise als eine Sendeantenne oder als eine Empfangsantenne eingesetzt werden. Der kreisförmigen Rahmen 703 sowie die Zuleitungen 705 können in oder auf einem Substrat angeordnet werden.

Fig. 7B zeigt eine Erregeranordnung mit einer Sendeantenne 707 eines Senders und einer Empfangsantenne 709 eines Empfängers, welche als Schleifenantennen gemäß

Fig. 7A gebildet sein können. Die Schleifenantennen 707, 709 sind beispielsweise derart angeordnet, dass die kreisförmigen Rahmen 703 oberhalb eines Blutgefäßes 711 angeordnet sind, wobei die Zuleitungen 705 quer zum Verlauf des Blutgefäßes 711, d.h. quer zur Blutflussrichtung, zeigen. Dadurch wird senderseitig ein Magnetfeld mit einer quer zum Verlauf des Blutgefäßes 711 weisenden Komponente H des Magnetfeldes erzeugt.

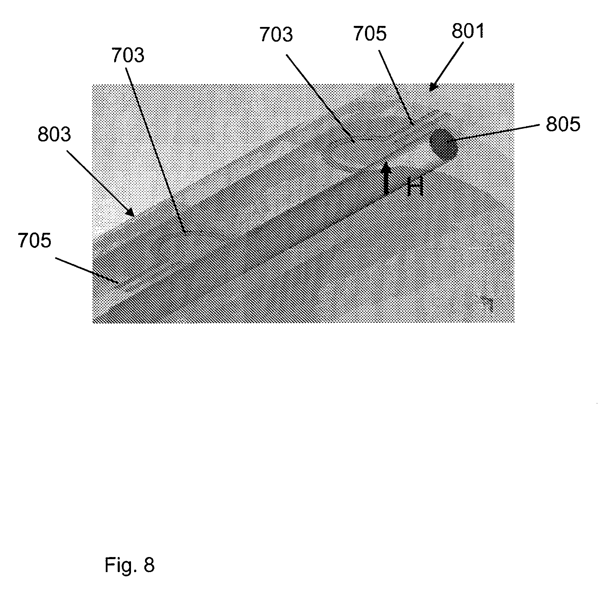

Fig. 8 zeigt eine Erregeranordnung einer Sendeantenne 801 eines Senders und einer Empfangantenne 803 eines Empfängers bezüglich eines Blutgefässes 805. Die Sendeantenne 801 und die Empfangsantenne 803 können beispielsweise Schleifenantennen mit der in Fig. 7A dargestellten Form sein. Sie sind beispielsweise derart angeordnet, dass die kreisrunden Rahmen 703 jeweils oberhalb des Blutgefässes 705 angeordnet sind und dass die Zuleitungen 705 voneinander weisend parallel zum Verlauf des Blutgefäßes 805 verlaufen. Dadurch wird eine senkrecht zum Verlauf des Blutgefäßes 805 weisende magnetische Feldkomponente H erzeugt, welche in Richtung einer Normalen der durch den kreisrunden Rahmen 803 aufgespannten Fläche zeigt.

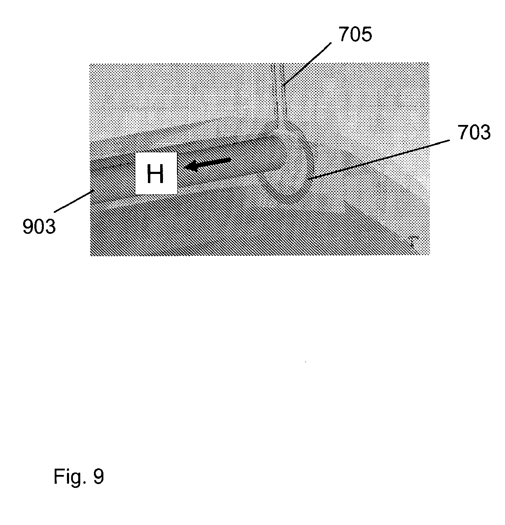

Fig. 9 zeigt eine Erregeranordnung mit einer Sendeantenne 901 eines Senders, welche beispielsweise die in Fig. 7A dargestellte Form einer Schleifenantenne aufweist. Die Sendeantenne 901 ist bezüglich eines Blutgefässes 903 beispielsweise derart angeordnet, dass eine Normale der durch den Rahmen 703 aufgespannten Fläche in Richtung des Verlaufes des Blutgefäßes 903 zeigt. Eine derartige Anordnung kann beispielsweise bei einem Knick des Blutgefäßes 903 realisiert werden. Dadurch wird eine in Richtung des Verlaufes des Blutgefäßes 903 weisende magnetische Feldkomponente H erzeugt.

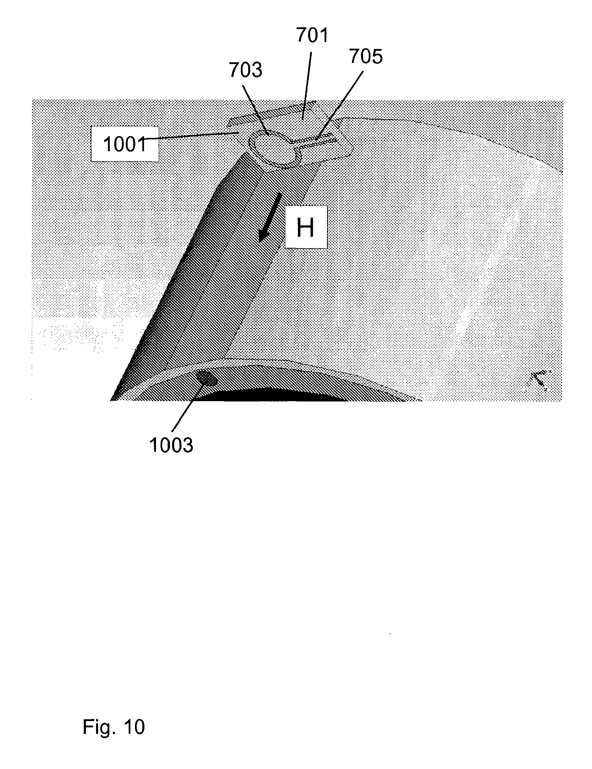

Fig. 10 zeigt eine Erregeranordnung mit einer Sendeantenne 701, welche beispielsweise eine Schleifenantenne mit der in Fig. 7A dargestellten Form ist und in einem Substrat 1001, beispielsweise Kunststoffsubstrat, angeordnet sein kann. Die Sendeantenne 701 ist oberhalb eines Blutgefässes 1003 derart angeordnet, dass eine Normale der durch den kreisförmigen Rahmen 703 aufgespannten Fläche in Richtung des Verlaufes des Blutgefäßes 1003 zeigt. Dadurch wird ein magnetisches Feld mit einer in Richtung des Verlaufes des Blutgefäßes 1003, d.h. in Blutflussrichtung, weisenden Feldkomponente H erzeugt.

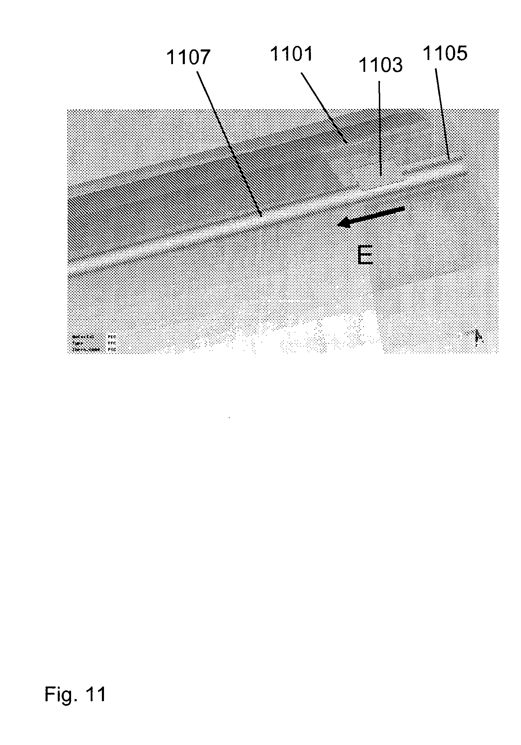

Fig. 11 zeigt eine Erregeranordnung mit einer Sendeantenne 1101, welche eine Patch-Antenne mit einer Patch-Antennenfläche 1103 und Zuleitungen 1105 sein kann. Die Patch-Antennenfläche 1103 ist beispielsweise oberhalb eines Blutgefäßes 1107 angeordnet, wodurch ein elektrisches Feld mit einer in Richtung eines Verlaufs des Blutgefäßes 1107, d.h. in Blutflussrichtung, weisenden elektrischen Feldkomponente E erzeugt wird.

Gemäß einer Ausführungsform ist der Verlustdetektor 107 ausgebildet, beispielsweise eine skalare oder eine vektorielle Messung oder eine Leistungsmessung durchzuführen. Zur Bestimmung der Verlustgrößen kann eine einfache spektroskopische Messung durchgeführt werden, bei der der Betrag des Messparameters S21 erfasst wird.

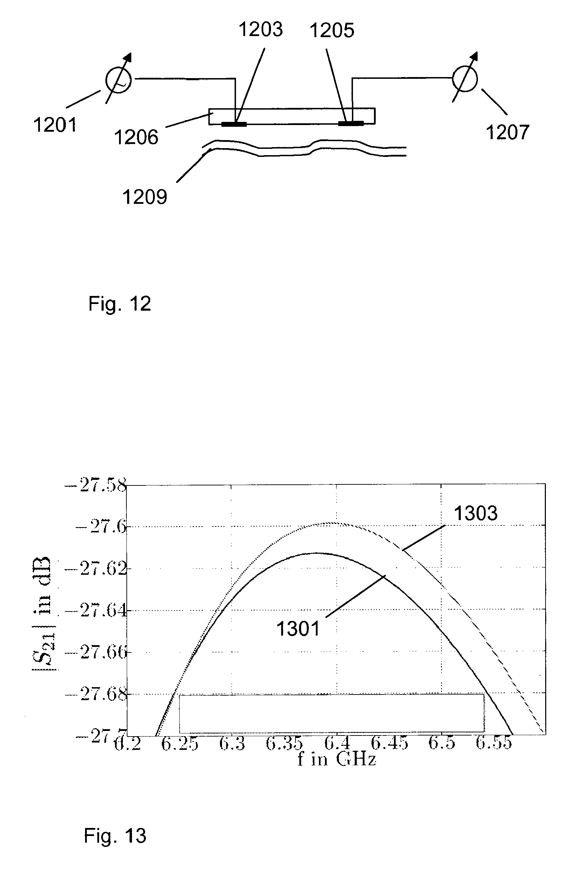

Die Messung von $|S_{21}|$ kann beispielsweise mittels der in Fig. 12 dargestellten Erfassungsvorrichtung durchgeführt werden. Die Erfassungsvorrichtung umfasst einen Sender mit einem Sendesignalgenerator 1201, welcher ein durchstimmbarer Oszillator sein kann. Ein Ausgang des Sendesignalgenerators 1201 ist mit einer Sendeantenne 1203 verbunden. Die Erfassungsvorrichtung umfasst ferner einen Empfänger mit einer Empfangsantenne 1205, deren Ausgang mit einem Verlustdetektor 1207 verbunden ist. Der Verlustdetektor kann beispielsweise einen Leistungsdetektor umfassen. Wie in Fig. 12 dargestellt sind die Sendeantenne 1203 und die Empfangsantenne 1205 oberhalb eines Blutgefäßes 1209 angeordnet. Der Sender kann Merkmale des Senders 101, der Empfänger kann Merkmale des Empfängers 105 und der Verlustdetektor 1207 kann Merkmale des Verlustdetektors 107 entsprechen.

Durch eine weitere Messung eines Betrags des Messparameters von S11 kann jedoch die Genauigkeit bei der Bestimmung der Verlustgrößen, d.h. der Verluste in dem Wellenleiter, noch weiter gesteigert werden. Die Verlustgrößen können beispielsweise auf der Basis der folgenden Formel bestimmt werden:

$$P_{Verlust} = 1 - |S_{11}|^2 - |S_{21}|^2,$$

wobei $P_{Verlust}$ die jeweilige Verlustgröße bezeichnet, und wobei $S_{11}$ den Eingangsreflexionsfaktor und $S_{21}$ den Vorwärtstransmissionsfaktor bezeichnen.

Zum Erfassen des Blutbildparameters, beispielsweise einer Konzentration von Blutzucker, können beispielsweise Frequenzverschiebungen von Absorptionslinien einer Wasserlösung mit Zucker untersucht werden.

Fig. 13 zeigt beispielsweise eine Frequenzverschiebung eines Absorptionsmaximums 1401 bei einer ersten Blutzuckerkonzentration im Vergleich zu einer Frequenzverschiebung eines Absorptionsmaximums 1403 bei einer zweiten Blutzuckerkonzentration, welche höher als die erste Blutzuckerkonzentration. Dabei wurde als Verlustgröße beispielhaft eine Transmission um 6 GHz erfasst.

Die Frequenzverschiebung des Absorptionsmaximums kann als ein Maß für einen Blutbildparameter, beispielsweise für einen Blutzuckerspiegel, aufgefasst werden. Durch eine Beobachtung von Frequenzverschiebungen bei mehreren Absorptionen einer Wasserlösung mit Zucker kann die Messzuverlässigkeit noch weiter erhöht werden.

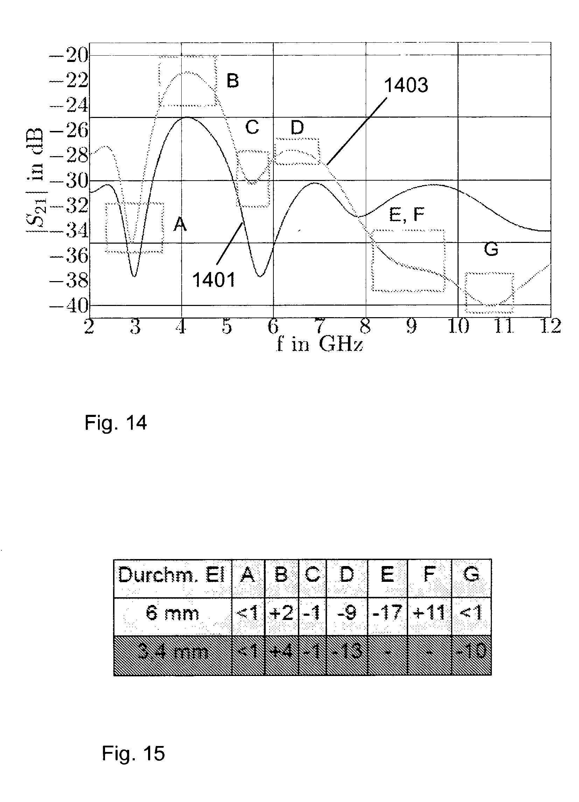

Fig. 14 zeigt beispielhaft ein breitbandiges Transmissionsverhalten von venösem Blut in einem Handgelenk. Dabei verdeutlichen die Verläufe 1401 und 1403 unterschiedliche Frequenzlagen von Absorptionslinien bei unterschiedlichen Blutzuckerkonzentrationen. Zur Erfassung des Blutbildparameters, wie beispielsweise der Konzentration des Blutzuckers, können beispielsweise gezielt Frequenzverschiebungen der Absorptionen A, B, C, D, E, F und G erfasst werden. So kann beispielsweise für jede Frequenz eines Absorptionsmaximums und/oder eines Absorptionsminimums eine Verschiebung in Richtung höherer oder niedriger Frequenzen je nach Blutzuckerspiegel beispielsweise in einem Frequenzbereich zwischen 2 GHz und 12 GHz beobachtet werden.

Fig. 15 zeigt beispielhaft Frequenzverschiebungen der in Fig. 14 dargestellten Absorptionen A, B, C, D, E, F und G für ein Blutgefäß mit 6 mm Durchmesser und für ein Blutgefäß mit 3,4 mm Durchmesser. Zu erkennen ist, dass die Absorptionen für eine Zuckerspiegelvariation Frequenzverschiebungen sowohl in positiver als auch in negativer Richtung aufweisen können. Die Erfassung von mehreren Absorptionen bzw. Absorptionslinien ermöglicht somit eine genauere Erfassung eines Blutbildparameters, beispielsweise des Blutzuckerspiegels.

Gemäß einer Ausführungsform kann der Sender 101 der in Fig. 1 gezeigten Erfassungsvorrichtung 100 ausgebildet sein, in das in Fig. 1 schematisch dargestellte Blutgefäß 103 ein erstes Sendesignal mit einer ersten Frequenz und ein zweites Sendesignal mit einer zweiten Frequenz einzukoppeln. Das erste Sendesignal und das zweite Sendesignal können beispielsweise gemeinsam ein Breitbandsignal ergeben. Der Sender 101 kann weiter dazu ausgebildet sein, das erste Sendesignal und das zweite Sendesignal sequenziell hintereinander in das Blutgefäß 103 einzukoppeln. Der Empfänger 105 kann analog ausgebildet sein, ein erstes Empfangssignal bei der ersten Frequenz und ein zweites Empfangssignal bei der zweiten Frequenz zu empfangen. Der Verlustdetektor kann vorgesehen sein, eine erste Verlustgröße auf der Basis des ersten Sendesignals und des ersten Empfangssignals sowie eine zweite Verlustgröße auf der Basis des zweiten Sendesignals und des zweiten Empfangssignals zu bestimmen. Der Prozessor kann vorgesehen sein, eine Relaxationszeitkonstante $\tau$ des Blutbildparameters in Abhängigkeit von der Frequenz mit der größeren Verlustgröße zu bestimmen. Bei Verwendung weiterer Sendesignal- und Empfangssignalpaare wird der Verlustdetektor 107 entsprechend weitere Verlustgrößen ermitteln.

Beispielsweise wird der Prozessor 109 die Relaxationszeitkonstante des Blutbildparameters in Abhängigkeit von der ersten Frequenz bestimmen, wenn die erste Verlustgröße größer als die zweite Verlustgröße ist. Entsprechend wird der Prozessor 109 die Relaxationszeitkonstante ($\tau$) des Blutbildparameters in Abhängigkeit von der zweiten Frequenz bestimmen, wenn die zweite Verlustgröße größer als die erste Verlustgröße ist. Dabei wird die Erkenntnis ausgenutzt, dass ein Blutbildparameter durch eine Erfassung der Relaxationszeitkonstante eines Blutbestandteils ermittelt werden kann. Ist der zu bestimmende Blutbildparameter beispielsweise eine Konzentration von Blutzucker in Blut, so ist eine Relaxationszeitkonstante einer Zucker enthaltenden Wasserlösung ein Maß für die Konzentration des Blutzuckers, d.h. für den Blutzuckerspiegel.

Ferner kann der Verlustdetektor eingerichtet sein, zur Bestimmung der jeweiligen Verlustgröße die komplexe Dielektrizitätszahl ε" zu ermitteln.

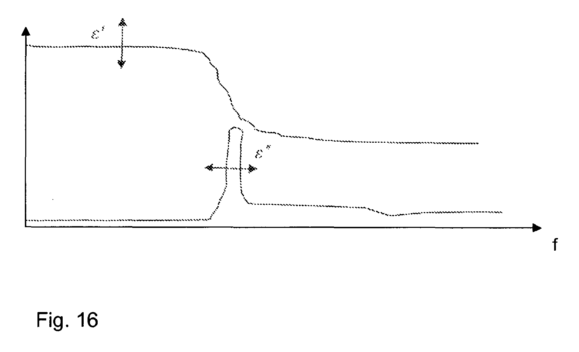

Hierzu zeigt die Fig. 16 ein Diagramm zur Illustrierung der reellen Dielektrizitätszahl ε' und der komplexen Dielektrizitätszahl ε" in Abhängigkeit der Frequenz f.

Dabei illustriert die Fig. 16, dass in dem Frequenzbereich, wo der Realteil ε' von dem höheren Niveau auf das niedrigere Niveau übergeht, die durch die komplexe Dielektrizitätszahl ε" repräsentierten Verluste steigen. Dieser Anstieg der Verluste wird in der Spektroskopie auch als Absorptionslinien bezeichnet. Der Effekt, der hierbei ausgenützt werden kann, ist der, dass sich die Frequenz, bei der die Überhöhung der Verluste - siehe lokales Maximum von ε" - mit der Konzentration des Zuckergehaltes verschiebt.

Beispielsweise der menschliche Körper besteht zu 80% aus Wasser. Wasser besitzt Absorptionslinien, z.B. bei 19 GHz und 50 GHz. Deren Verstimmung kann bestimmt werden und auf den Zuckergehalt abgebildet werden. Die Verstimmung der Resonanzfrequenz bei ε" ist - wie Fig. 16 illustriert - leichter detektierbar als die Plateauveränderung von ε'. Insbesondere verschieben Variationen in der Ankopplung die Frequenz des Maximums von ε" vorteilhafterweise nicht. Damit ist eine Bestimmung der Zuckerkonzentration aus der Beobachtung von ε" deutlich weniger fehleranfällig als die Beobachtung von ε' bzw. dessen Niveauänderungen.

Weil bei einer Vielzahl von Stoffen solche Kurven wie die der Fig. 16 überlagert sind, ist eine Separation der Stoffe durch eine Beobachtung der imaginären Dielektrizitätszahl ε" einfacher durchführbar, da jedem Stoff ein bestimmtes Absorptionsmaximum zugeordnet werden kann. Bei der reellen Dielektriziätszahl ε' kann jedoch nur die Summe aller reellen Dielektrizitätszahlen ε' aller beteiligten Stoffe beobachtet werden.

Wie oben bereits ausgeführt, ist der Prozessor 109 dazu ausgebildet, die Relaxationskonstante $\tau$ des Blutbildparameters in Abhängigkeit von der Frequenz mit der größeren oder maximalen Verlustgröße zu bestimmen. Weiter ist der Prozessor 109 dazu ausgebildet, den Blutbildparameter, wie die Glucosekonzentration im Blut, in Abhängigkeit der bestimmten Relaxationszeitkonstanten zu ermitteln.

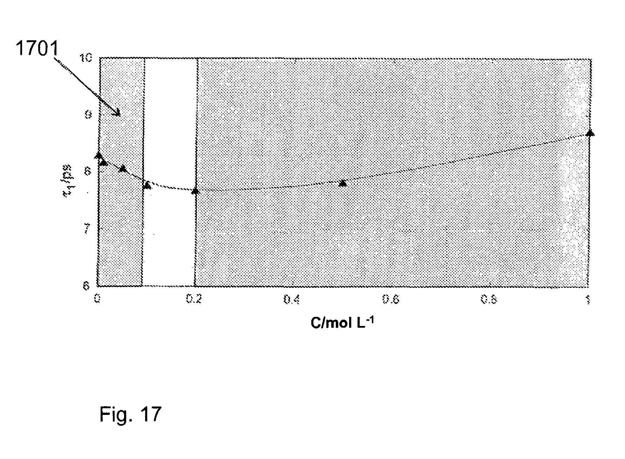

Dazu zeigt die Fig. 17 ein Diagramm zur Illustrierung eines Zusammenhangs zwischen der Relaxationszeitkonstanten $\tau$ und der Glucosekonzentration $C/\text{mol L}^{-1}$ im Blut. Dabei zeigt der mit dem Bezugszeichen 1701 referenzierte Bereich in der Fig. 17 einen kritischen Blutzuckerbereich.

Ferner ist der Prozessor 109 insbesondere dazu ausgebildet, die Relaxationszeitkonstante ($\tau$) auf der Basis der Formel $\tau = \dfrac{1}{2\pi f_A}$ zu berechnen, wobei $f_A$ die Frequenz bezeichnet, bei welcher die ermittelte Verlustgröße maximal ist.

Vorteilhafterweise ist dann der Prozessor 109 dazu ausgebildet, die Frequenz zu bestimmen, bei welcher der Imaginärteil der komplexen Dielektrizitätszahl ε" maximal ist, und die Relaxationszeitkonstante ($\tau$) in Abhängigkeit der bestimmten Frequenz zu ermitteln ist. Diese bestimmte Frequenz nutzt der Prozessor 109 dann zur Bestimmung des Blutbildparameters, wie der Glucosekonzentration.

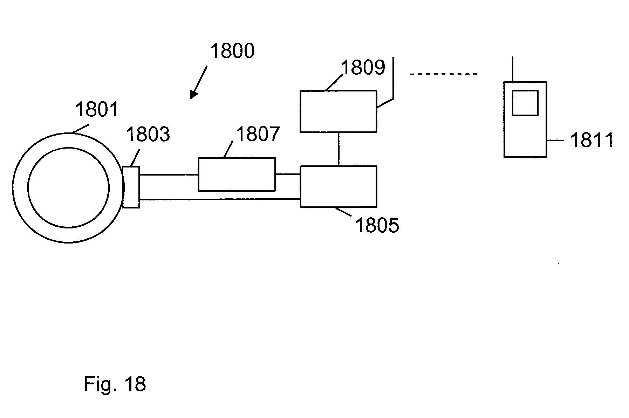

Die Fig. 18 zeigt ein schematisches Blockdiagramm einer Erfassungsvorrichtung 1800. Die Erfassungsvorrichtung 1800 hat ein Armband 1801, einen an dem Armband 1801 befestigten Sensor-Array 1803, einen Mikroprozessor 1805, einen Mikrowellenschaltkreis 1807 zur Generierung der Sendesignale und eine Kommunikationsvorrichtung 1809.

Der Sensor-Array 1803 hat beispielsweise einen Mikrowellensensor, einen Temperatursensor und einen Feuchtigkeitssensor.

Der Mikroprozessor 1805 ist beispielsweise wie der Prozessor 109 der Fig. 1 ausgebildet.

Die Kommunikationsvorrichtung 1809 ist dazu eingerichtet, eine Kommunikationsverbindung für die Erfassungsvorrichtung 1800 zu einer weiteren Kommunikationsvorrichtung 1811 bereitzustellen. Die Kommunikationsvorrichtung 409 umfasst beispielsweise eine Bluetooth-Schnittstelle. Die weitere Kommunikationsvorrichtung 1811 ist beispielsweise ein Mobilfunkgerät, ein Smartphone oder eine GPS-basierte Einrichtung.

Die Messungen oder Erfassungen von Blutbildparametern können durch ein Armband mit einer Erfassungsvorrichtung der vorgenannten Erfassungsvorrichtungen reproduzierbar bereitgestellt werden, wenn das Armband mit der Erfassungsvorrichtung mit einem vorbestimmten oder vorgegebenen Anpressdruck während der Messungen auf den Arm gedrückt wird. Zum Bereitstellen des vorgegebenen Anpressdruckes wird das Armband zusätzlich zu der Erfassungsvorrichtung mit einer Einstellvorrichtung ausgestattet. Die Einstellvorrichtung ist derart ausgebildet, dass sie den vorgegebenen oder vorbestimmten Anpressdruck zumindest während des Erfassens des Blutbildparameters durch die Erfassungsvorrichtung einstellen kann.

Das Armband mit der Erfassungsvorrichtung und der Einstellvorrichtung ist dazu eingerichtet, am Arm des Patienten, insbesondere im Bereich seines Handgelenkes, angelegt zu werden.

Dieser Ort des Handgelenkes bedingt insbesondere keine Beeinträchtigung der Bewegungsfreiheit des Patienten. Des Weiteren ist dieser Ort bei den Patienten bereits für herkömmliche Blutdruckmessgeräte etabliert. Ein weiterer Vorteil des Anlegens des Armbandes an das Handgelenk liegt darin, dass an dieser Stelle üblicherweise der Puls ertastet wird, die Haut dünn ist und somit Fehlerquellen reduziert werden können. Ferner werden durch das Anpressen des Armbandes während des Erfassens des Blutbildparameters Luftspalte vermieden, welche eine mikrowellentechnische Anpassung durch die Erfassungsvorrichtung verändern könnten. Durch das Anpressen des Armbandes mit dem vorgegebenen Anpressdruck kann sich das Armband an die Anatomie des jeweiligen Patienten anpassen.

Durch die Verwendung des Armbandes mit der Erfassungsvorrichtung und der Einstellvorrichtung kann der Blutbildparameter kontinuierlich überwacht werden. Ein Beispiel für einen solchen Blutbildparameter ist - wie oben ausgeführt - die Blutzuckerkonzentration. Durch die Möglichkeit der kontinuierlichen Überwachung der Blutzuckerkonzentration wird eine Bestimmung der Verzögerungszeit zwischen der Nahrungsaufnahme und dem Blutzuckeranstieg ermöglicht. Weiter kann auf Variationen im Tagesablauf des Patienten sehr schnell reagiert werden. Insbesondere kann bei einer Überzuckerung oder einer Unterzuckerung zeitnah ein Alarm ausgelöst werden.

Gemäß einem weiteren Aspekt wird ein Armband vorgeschlagen, welches eine Erfassungsvorrichtung zum Erfassen eines Blutbildparameters von Blut in einem Blutgefäß des Armes und eine Einstellvorrichtung zum Einstellen eines vorgegebenen Anpressdruckes des Armbandes auf den Arm aufweist. Die Erfassungsvorrichtung kann die Merkmale der vorgenannten Erfassungsvorrichtungen aufweisen.

Gemäß einer Ausführungsform wird die Messung breitbandig statt schmalbandig durchgeführt. Die Sendesignale können beispielsweise mittels eines Frequenz-Sweeps oder als Teilsignal eines breitbandigen Sendesignals in das Blutgefäß eingekoppelt werden. Durch die bevorzugt vektorielle Erfassung der S-Parameter kann nun die komplexe Dielektrizitätszahl und nicht nur deren Realteil ausgewertet werden. Durch Beobachtung von Frequenzverschiebungen von mehreren Absorptionslinien kann die Bestimmung des Blutbildparameters genauer durchgeführt werden. Bevorzugt wird dieser mittels einer Zweitormessung und nicht mittels einer Eintormessung durchgeführt.

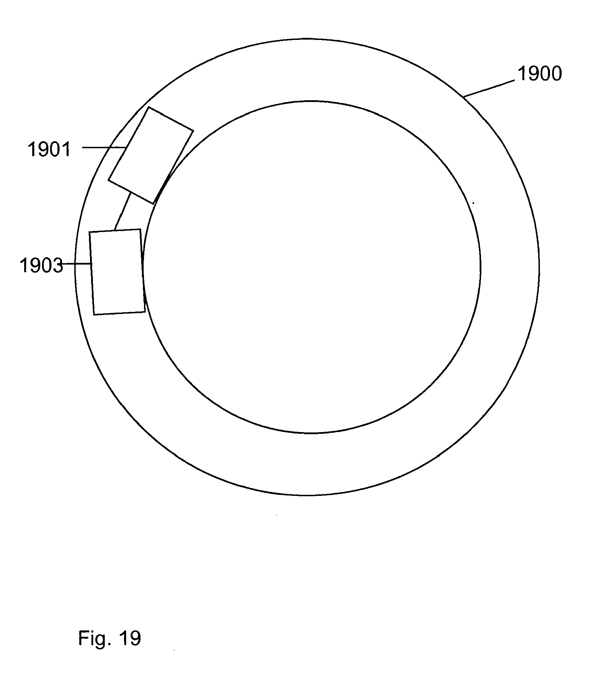

Die Fig. 19 zeigt ein Blockdiagramm eines Ausführungsbeispiels eines Armbandes 1900 mit einer Erfassungsvorrichtung 1901 und einer Einstellvorrichtung 1903. Die Erfassungsvorrichtung 1901 ist zum Erfassen eines Blutbildparameters von Blut in einem Blutgefäß des Armes eingerichtet. Ein Beispiel für den zu erfassenden Blutbildparameter ist die Glukosekonzentration in dem Blut.

Die Einstellvorrichtung 1903 ist zum Einstellen eines vorgebbaren Anpressdruckes des Armbandes 1900 auf den Arm eingerichtet. Durch Einstellen des vorgegebenen Anpressdruckes des Armbandes 1900 kann die Einstellvorrichtung 1903 reproduzierbare Erfassungen des Blutbildparameters durch die Erfassungsvorrichtung 1901 sicherstellen. Dazu ist die Einstellvorrichtung 1903 insbesondere dazu eingerichtet, den Anpressdruck des Armbandes 1900 während des Erfassens des Blutbildparameters durch die Erfassungsvorrichtung 1901 auf den vorgebbaren Anpressdruck einzustellen.

Das Armband 1900 ist insbesondere als ein aufblasbares Armband 1900 ausgebildet. Dabei hat die Einstellvorrichtung 1903 insbesondere eine Luftpumpe, welche dazu ausgebildet ist, das Armband 1900 zum Einstellen des vorgegebenen Anpressdruckes aufzublasen.

Im Detail umfasst die Erfassungsvorrichtung 1901 insbesondere Elektroden, welche zur Einkopplung zumindest eines Hochfrequenzsignals in das Blutgefäß eingerichtet sind. Das Hochfrequenzsignal ist dazu ausgebildet, einen Parameter zur Erfassung des Blutbildparameters zu liefern. Ein Beispiel für einen solchen Parameter bildet die Relaxationszeitkonstante $\tau$ des Blutbildparameters. Dabei ist die Einstellvorrichtung 1903 insbesondere dazu ausgebildet, den Anpressdruck der Elektroden auf den Arm auf den vorgegebenen Anpressdruck einzustellen.

Des Weiteren kann die Einstellvorrichtung 1903 derart ausgebildet werden, dass sie die Anpresskräfte des Armbandes 1900 während des Erfassens des Blutbildparameters durch die Erfassungsvorrichtung 1901 gleichmäßig auf den Arm verteilt. Ferner ist die Einstellvorrichtung 1903 vorzugsweise derart ausgebildet, dass sie ein gleichmäßiges Anliegen des Armbandes 1900 während des Erfassens des Blutbildparameters durch die Erfassungsvorrichtung 1901 sicherstellt.

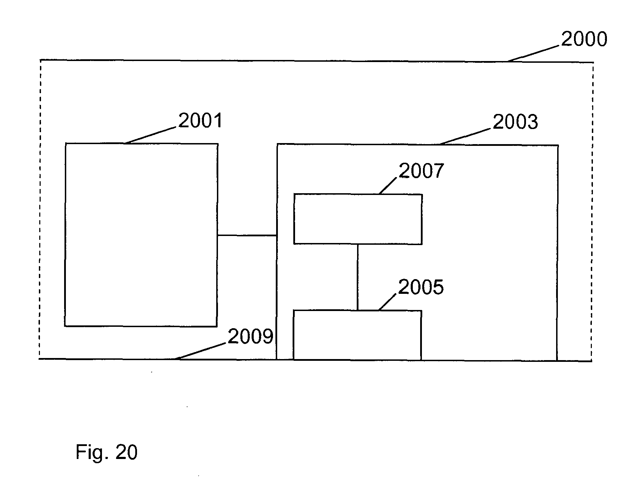

Die Fig. 20 zeigt ein Blockschaltbild eines Ausschnittes eines Ausführungsbeispiels eines Armbandes 2000. Das Armband 2000 hat eine Erfassungsvorrichtung 2001 und eine Einstellvorrichtung 2003. Die Erfassungsvorrichtung 2001 und die Einstellvorrichtung 2003 sind zumindest wie die Erfassungsvorrichtung 1901 und die Einstellvorrichtung 1903 der Fig. 19 ausgebildet. Des Weiteren hat die Einstellvorrichtung 2003 der Fig. 20 eine Sensoreinrichtung 2005 und eine Steuereinrichtung 2007. Die Sensoreinrichtung 2005 ist dazu eingerichtet, einen aktuellen Anpressdruck des Armbandes 2000 auf den Arm zu messen. In Abhängigkeit des gemessenen aktuellen Anpressdruckes stellt die Steuereinrichtung 2007 den vorgegebenen Anpressdruck auf den Arm ein.

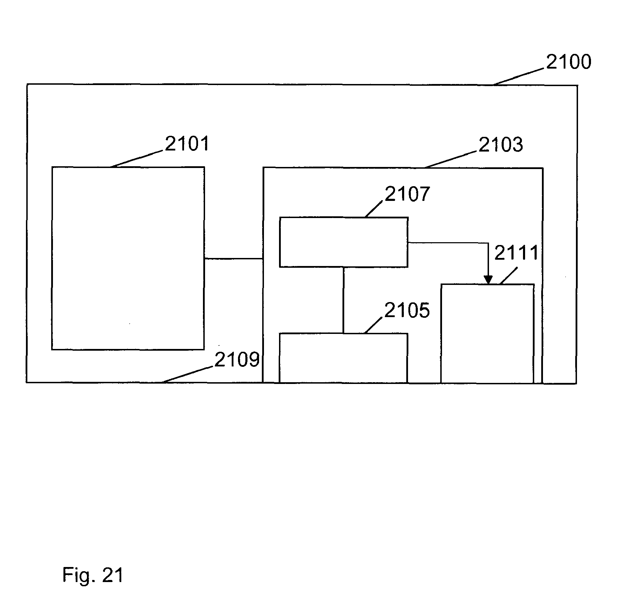

Die Fig. 21 zeigt ein Blockschaltbild eines Ausschnittes eines weiteren Ausführungsbeispiels eines Armbandes 2100. Das Armband 2100 hat eine Erfassungsvorrichtung 2101 und eine Einstellvorrichtung 2103. Die Einstellvorrichtung 2103 hat eine Sensoreinrichtung 2105, eine Steuereinrichtung 2107 und eine Luftpumpe 2111. Die Sensoreinrichtung 2105 misst einen aktuellen Anpressdruck des Armbandes 2100 auf den Arm. Die Steuereinrichtung 2107 stellt ein Steuersignal in Abhängigkeit des gemessenen aktuellen Anpressdruckes bereit. Mittels des bereitgestellten Steuersignals wird die Luftpumpe 2111 zum Aufblasen des Armbandes 2100 gesteuert.

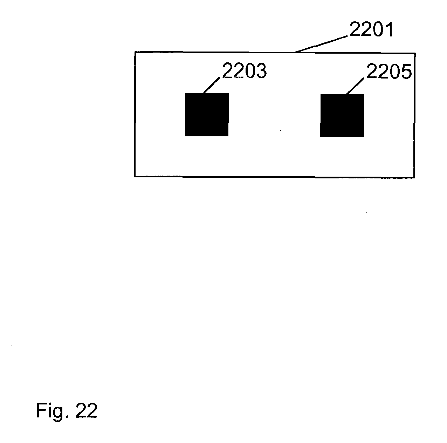

In Fig. 22 ist ein schematisches Blockdiagramm einer Anordnung 2200 der Elektroden, d.h. Antennen 2203, 2205 der Erfassungsvorrichtung zum Erfassen eines Blutbildparameters von Blut in einem Blutgefäß des Armes dargestellt

Ohne Einschränkung der Allgemeinheit zeigt die Anordnung 2200 nur zwei Elektroden 2203 und 2205. Die Anordnung 2200 ist insbesondere Teil der Erfassungsvorrichtung und beispielsweise als eine Platte mit beispielhaften Abmessungen von 5cm auf 2cm ausgestaltet. Die Elektroden 2203, 2205 haben beispielsweise eine Grundfläche von 5mm auf 5mm. Der Abstand der Elektroden 2203, 2205 beträgt beispielsweise 1 bis 2cm. Damit wird zum einen eine genügend starke Transmission erzielt und zum anderen wird eine genügend große Eindringtiefe in den Körper sichergestellt.

**Patentansprüche**

1. Erfassungsvorrichtung zum Erfassen eines Blutbildparameters von Blut in einem Blutgefäß, mit:

einem Sender (101) mit einer Anzahl von Sendeantennen (203) zum Aussenden von zumindest einem Sendesignal;
einem Empfänger (105) mit einer Anzahl von Empfangsantennen (207) zum Empfangen von zumindest einem Empfangssignal;
einem Prozessor (109), welcher ausgebildet ist, eine erste Erfassungskonfiguration umfassend eine Sendeantenne der Anzahl der Sendeantennen (203) und eine Empfangsantenne der Anzahl der Empfangsantennen (207) auszuwählen, und eine zweite Erfassungskonfiguration umfassend eine Sendeantenne der Anzahl der Sendeantennen (203) und eine Empfangsantenne (207) der Anzahl von Empfangsantennen auszuwählen;
einem Verlustdetektor (107), welcher ausgebildet ist,
bei Auswahl der ersten Erfassungskonfiguration zum Aussenden eines Sendesignals eine erste Verlustgröße auf der Basis des Sendesignals und eines Empfangssignals zu erfassen, und
bei Auswahl der zweiten Erfassungskonfiguration zum Aussenden eines Sendesignals eine zweite Verlustgröße auf der Basis des Sendesignals und eines Empfangssignals zu erfassen wobei
der Prozessor ausgebildet ist, die Erfassungskonfiguration mit der geringeren Verlustgröße zum Erfassen des Blutbildparameters auszuwählen.

2. Erfassungsvorrichtung nach Anspruch 1, wobei, der Sender (101) bei Auswahl der ersten Erfassungskonfiguration ausgebildet ist, das Sendesignal mittels der Sendeantenne der ersten Erfassungskonfiguration auszusenden, und wobei der Empfänger (105) bei Auswahl der ersten Erfassungskonfiguration ausgebildet ist, das Empfangssignal mittels der Empfangsantenne der ersten Erfassungskonfiguration zu empfangen, wobei
der Sender (101) bei Auswahl der zweiten Erfassungskonfiguration ausgebildet ist, das Sendesignal mittels der Sendeantenne der zweiten Erfassungskonfiguration auszusenden, und wobei der Empfänger (105) bei Auswahl der zweiten Erfassungskonfiguration ausgebildet ist, das Empfangssignal mittels der Empfangsantenne der ersten Erfassungskonfiguration zu empfangen, und wobei
der Verlustdetektor (107) ausgebildet ist, die erste Verlustgröße auf der Basis des Sendesignals und des Empfangssignals der ersten Erfassungskonfiguration zu erfassen, und die zweite Verlustgröße auf der Basis des Sendesignals und des Empfangssignals der zweiten Erfassungskonfiguration zu erfassen.

3. Erfassungsvorrichtung nach einem der vorstehenden Ansprüche, wobei der Prozessor (109) ausgebildet ist, die erste Verlustgröße mit der zweiten Verlustgröße zu vergleichen, um die geringere Verlustgröße der beiden Verlustgrößen zu bestimmen.

4. Erfassungsvorrichtung nach einem der vorstehenden Ansprüche, die ferner eine Schaltmatrix (211), insbesondere eine durch den Prozessor (109) steuerbare Schaltmatrix (211), umfasst, welche ausgebildet ist, jeweils einen Ausgang einer Empfangsantenne der Anzahl der Empfangsantennen mit dem Verlustdetektor (107) zu verbinden.

5. Erfassungsvorrichtung nach einem der vorstehenden Ansprüche, wobei der Sender (101) einen Sendesignalgenerator (202) umfasst, und wobei ein Ausgang des Sendesignalgenerators (202) mittels einer Schaltmatrix (209), insbesondere mittels einer durch den Prozessor (109) steuerbaren Schaltmatrix (209), mit jeweils einer Sendeantenne der Anzahl der Sendeantennen verbindbar ist.

6. Erfassungsvorrichtung nach einem der vorstehenden Ansprüche, wobei der Sender (101) ausgebildet ist, bei der Verwendung der ersten Erfassungskonfiguration und bei der Verwendung der zweiten Erfassungskonfiguration Sendesignale gleicher Frequenz, insbesondere einer Frequenz in einem Frequenzbereich zwischen 1 GHz und 15 GHz, oder gleicher Mode, insbesondere transversal-elektrischer Mode oder transversal-magnetischer Mode, auszusenden.

7. Erfassungsvorrichtung nach einem der vorstehenden Ansprüche, wobei der Sender (101) zumindest zwei Sendeantennen (203) oder wobei der Empfänger zumindest zwei Empfangsantennen (207), insbesondere Dipolantennen oder Rahmenantennen oder Patch-Antennen, umfasst.

8. Erfassungsvorrichtung nach einem der vorstehenden Ansprüche, wobei der Verlustdetektor (107) ein Netzwerkanalysator, insbesondere ein vektorieller oder ein skalarer Netzwerkanalysator, oder ein Leistungsdetektor ist.

9. Erfassungsvorrichtung nach einem der vorstehenden Ansprüche, wobei zum Erfassen des Blutbildparameters
der Sender (101) ausgebildet ist, unter Verwendung der Sendeantenne der ausgewählten Erfassungskonfiguration ein erstes Sendesignal mit einer ersten Frequenz und ein zweites Sendesignal mit einer zweiten Frequenz in das Blutgefäß einzukoppeln;
der Empfänger (105) ausgebildet ist, unter Verwendung der Empfangsantenne der ausgewählten Erfassungskonfiguration ein erstes Empfangssignal bei der ersten Frequenz und ein zweites Empfangssignal bei der zweiten Frequenz zu empfangen;
der Verlustdetektor (107) ausgebildet ist, eine erste Verlustgröße auf der Basis des ersten Sendesignals und des ersten Empfangssignals bei der ersten Frequenz zu bestimmen, und eine zweite Verlustgröße auf der Basis des zweiten Sendesignals und des zweiten Empfangssignals bei der zweiten Frequenz zu bestimmen; und
der Prozessor (109) ausgebildet ist, eine erste Frequenzverschiebung der ersten Verlustgröße relativ zu einer ersten Referenzverlustgröße zu bestimmen, eine zweite Frequenzverschiebung der zweiten Verlustgröße relativ zu einer zweiten Referenzverlustgröße zu ermitteln, und den Blutbildparameter auf der Basis der ersten Frequenzverschiebung und der zweiten Frequenzverschiebung zu ermitteln.

10. Erfassungsvorrichtung nach einem der vorstehenden Ansprüche, wobei zum Erfassen des Blutbildparameters
der Sender (101) ausgebildet ist, unter Verwendung der Sendeantenne der ausgewählten Erfassungskonfiguration ein erstes Sendesignal mit einer ersten Frequenz und eines zweites Sendesignal mit einer zweiten Frequenz in das Blutgefäß einzukoppeln;
der Empfänger (105) ausgebildet ist, unter Verwendung der Empfangsantenne der ausgewählten Erfassungskonfiguration ein erstes Empfangssignal bei der ersten Frequenz und ein zweites Empfangssignal bei der zweiten Frequenz zu empfangen; und wobei
der Prozessor (109) ausgebildet ist, eine erste Verlustgröße auf der Basis des ersten Sendesignals und des ersten Empfangssignals zu ermitteln, eine zweite Verlustgröße auf der Basis des zweiten Sendesignals und des zweiten Empfangssignals zu ermitteln, eine Relaxationszeitkonstante eines Blutbestandteils in Abhängigkeit von der Frequenz mit der größeren Verlustgröße zu bestimmen, und den Blutbildparameter auf der Basis eines vorbekannten Zusammenhanges zwischen der Relaxationszeitkonstanten und dem Blutbildparameter zu ermitteln.

11. Erfassungsvorrichtung nach einem der vorstehenden Ansprüche, wobei der Verlustdetektor (107) ausgebildet ist, zur Bestimmung der ersten Verlustgröße und der zweiten Verlustgröße eine Zweitormessung durchzuführen, insbesondere mittels der Zweitormessung jeweils einen Vorwärtstransmissionsfaktor $S_{21}$ zu bestimmen.

12. Erfassungsvorrichtung nach einem der vorstehenden Ansprüche, wobei der Verlustdetektor (107) ausgebildet ist, die erste Verlustgröße und die zweiten Verlustgröße jeweils auf der Basis der folgenden Formel zu bestimmen:

$$P_{Verlust} = 1 - \left| S_{11} \right|^2 - \left| S_{21} \right|^2,$$

wobei $P_{Verlust}$ die jeweilige Verlustgröße bezeichnet, und wobei $S_{11}$ den Eingangsreflexionsfaktor und $S_{21}$ den Vorwärtstransmissionsfaktor bezeichnen.

13. Erfassungsvorrichtung nach einem der vorstehenden Ansprüche, wobei der Sender (101) ausgebildet ist, das Sendesignal als eine Mode oder einen Wellentyp, insbesondere als eine transversal-elektrische (TE) Welle oder eine transversal-magnetische (TM) Welle oder eine transversal-elektromagnetische (TEM) Welle oder eine HE-Welle einzukoppeln, insbesondere tangential oder transversal bezüglich eines Verlaufes des Blutgefäßes oder bezüglich einer Blutflussrichtung einzukoppeln.

14. Erfassungsvorrichtung nach einem der vorstehenden Ansprüche, wobei der Blutbildparameter eine Konzentration eines Blutbestandteils, insbesondere Zuckers wie Glucose, oder Sauerstoffs, ist.

15. Verfahren zum Erfassen eines Blutbildparameters von Blut in einem Blutgefäß unter Verwendung eines Senders mit einer Anzahl von Sendeantennen zum Aussenden von zumindest einem Sendesignal und eines Empfängers mit einer Anzahl von Empfangsantennen zum Empfangen von zumindest einem Empfangssignal, mit:

  Auswählen einer ersten Erfassungskonfiguration umfassend eine Sendeantenne der Anzahl der Sendeantennen und eine Empfangsantenne der Anzahl von Empfangsantennen auszuwählen;

bei Auswahl der ersten Erfassungskonfiguration zum Aussenden eines Sendesignals, Erfassen einer ersten Verlustgröße auf der Basis des Sendesignals und eines Empfangssignals, Auswählen einer zweiten Erfassungskonfiguration umfassend eine Sendeantenne der Anzahl der Sendeantennen und eine Empfangsantenne der Anzahl von Empfangsantennen; bei Auswahl der zweiten Erfassungskonfiguration zum Aussenden eines Sendesignals, Erfassen einer zweite Verlustgröße auf der Basis des Sendesignals und eines Empfangssignals; und Auswählen die Erfassungskonfiguration mit der geringeren Verlustgröße zum Erfassen des Blutbildparameters.

## Claims

1. Detecting device for detecting a blood count parameter of blood in a blood vessel, comprising:

   a transmitter (101) with a number of transmission antennas (203) for emitting at least one transmission signal;
   a receiver (105) with a number of reception antennas (207) for receiving at least one reception signal;
   a processor (109) embodied to select a first detecting configuration, comprising one transmission antenna of the number of transmission antennas (203) and one reception antenna of the number of reception antennas (207), and to select a second detecting configuration, comprising one transmission antenna of the number of transmission antennas (203) and one reception antenna (207) of the number of reception antennas;
   a loss detector (107) configured to
   detect a first loss variable on the basis of the transmission signal and a reception signal when the first detecting configuration for emitting a transmission signal is selected and
   detect a second loss variable on the basis of the transmission signal and a reception signal when the second detecting configuration for emitting a transmission signal is selected, wherein
   the processor is embodied to select the detecting configuration with the smaller loss variable for detecting the blood count parameter.

2. Detecting device according to Claim 1, wherein the transmitter (101) is embodied to emit the transmission signal by means of the transmission antenna of the first detecting configuration when the first detecting configuration is selected and wherein the receiver (105) is embodied to receive the reception signal by means of the reception antenna of the first detecting configuration when the first detecting configuration is selected, wherein
the transmitter (101) is embodied to emit the transmission signal by means of the transmission antenna of the second detecting configuration when the second detecting configuration is selected and wherein the receiver (105) is embodied to receive the reception signal by means of the reception antenna of the first detecting configuration when the second detecting configuration is selected, and wherein
the loss detector (107) is embodied to detect the first loss variable on the basis of the transmission signal and the reception signal of the first detecting configuration and to detect the second loss variable on the basis of the transmission signal and the reception signal of the second detecting configuration.

3. Detecting device according to one of the preceding claims, wherein the processor (109) is embodied to compare the first loss variable to the second loss variable in order to determine the smaller loss variable of the two loss variables.

4. Detecting device according to one of the preceding claims, which furthermore comprises a switching matrix (211), in particular a switching matrix (211) controllable by the processor (109), which switching matrix is embodied to connect in each case an output of a reception antenna of the number of reception antennas with the loss detector (107).

5. Detecting device according to one of the preceding claims, wherein the transmitter (101) comprises a transmission signal generator (202) and wherein an output of the transmission signal generator (202) is connectable in each case to one transmission antenna of the number of transmission antennas by means of a switching matrix (209), in particular by means of a switching matrix (209) controllable by the processor (109).

6. Detecting device according to one of the preceding claims, wherein the transmitter (101) is embodied to emit transmission signals with the same frequency, in particular a frequency in a frequency range between 1 GHz and 15 GHz, or with the same mode, in particular a transverse electric mode or transverse magnetic mode, when using the first detecting configuration and when using the second detecting configuration.

7. Detecting device according to one of the preceding claims, wherein the transmitter (101) comprises at least two transmission antennas (203) or wherein the receiver comprises at least two reception antennas (207), in particular dipole antennas or frame antennas or patch antennas.

8. Detecting device according to one of the preceding claims, wherein the loss detector (107) is a network analyser, in particular a vector or scalar network analyser, or a power detector.

9. Detecting device according to one of the preceding claims, wherein, in order to detect the blood count parameter,
the transmitter (101) is embodied to couple a first transmission signal at a first frequency and a second transmission signal at a second frequency into the blood vessel using the transmission antenna in the selected detecting configuration;
the receiver (105) is embodied to receive a first reception signal at the first frequency and a second reception signal at the second frequency using the reception antenna of the selected detecting configuration;
the loss detector (107) is embodied to determine a first loss variable on the basis of the first transmission signal and the first reception signal at the first frequency and to determine a second loss variable on the basis of the second transmission signal and the second reception signal at the second frequency; and
the processor (109) is embodied to determine a first frequency shift of the first loss variable relative to a first reference loss variable, to establish a second frequency shift of the second loss variable relative to a second reference loss variable and to establish the blood count parameter on the basis of the first frequency shift and the second frequency shift.

10. Detecting device according to one of the preceding claims, wherein, in order to detect the blood count parameter,
the transmitter (101) is embodied to couple a first transmission signal at a first frequency and a second transmission signal at a second frequency into the blood vessel using the transmission antenna in the selected detecting configuration;
the receiver (105) is embodied to receive a first reception signal at the first frequency and a second reception signal at the second frequency using the reception antenna in the selected detecting configuration; and wherein
the processor (109) is embodied to establish a first loss variable on the basis of the first transmission signal and the first reception signal, to establish a second loss variable on the basis of the second transmission signal and the second reception signal, to determine a relaxation time constant of a blood constituent depending on the frequency with the greater loss variable and to establish the blood count parameter on the basis of a predefined relationship between the relaxation time constant and the blood count parameter.

11. Detecting device according to one of the preceding claims, wherein the loss detector (107) is embodied to carry out a two-port measurement, in particular to determine a forward transmission factor $S_{21}$ in each case by means of the two-port measurement, in order to determine the first loss variable and the second loss variable.

12. Detecting device according to one of the preceding claims, wherein the loss detector (107) is embodied to determine the first loss variable and the second loss variable on the basis of the following formula in each case:

$$P_{loss} = 1 - |S_{11}|^2 - |S_{21}|^2,$$

where $P_{loss}$ denotes the respective loss variable and where $S_{11}$ denotes the input reflection factor and $S_{21}$ denotes the forward transmission factor.

13. Detecting device according to one of the preceding claims, wherein the transmitter (101) is embodied to couple the transmission signal in as a mode or a wave type, in particular as a transverse electric (TE) wave or as a transverse magnetic (TM) wave or as a transverse electromagnetic (TEM) wave or an HE wave, in particular tangentially or transversely with respect to an extent of the blood vessel or with respect to a blood flow direction.

14. Detecting device according to one of the preceding claims, wherein the blood count parameter is a concentration of a blood constituent, in particular sugar such as glucose, or a concentration of oxygen.

15. Method for detecting a blood count parameter of blood in a blood vessel, using a transmitter with a number of transmission antennas for emitting at least one transmission signal and a receiver with a number of reception antennas for receiving at least one reception signal, comprising:

    selecting a first detecting configuration comprising a transmission antenna of the number of transmission antennas and a reception antenna of the number of reception antennas;
    detecting a first loss variable on the basis of the transmission signal and a reception signal when the first detecting configuration for emitting a transmission signal is selected,
    selecting a second detecting configuration comprising a transmission antenna of the number of transmission antennas and a reception antenna of the number of reception antennas;
    detecting a second loss variable on the basis of the transmission signal and a reception signal when the second detecting configuration for emitting a transmission signal is selected; and selecting the detecting configuration with the smaller loss variable for detecting the blood count parameter.

## Revendications

1.  Dispositif de saisie destiné à saisir un paramètre d'imagerie du sang dans un vaisseau sanguin, le dispositif présentant:

    un émetteur (101) qui présente plusieurs antennes émettrices (203) pour émettre au moins un signal d'émission;
    un récepteur (105) présentant plusieurs antennes de réception (207) pour recevoir au moins un signal de réception;
    un processeur (109) configuré pour sélectionner une première configuration de saisie qui comprend une des antennes d'émission (203) et une des antennes de réception (207) et une deuxième configuration de saisie qui comprend une des antennes d'émission (203) et une des antennes de réception (207);
    un détecteur de perte (107) configuré pour saisir une première grandeur de perte sur la base du signal d'émission et d'un signal de réception lors de la sélection de la première configuration de saisie qui émet un signal d'émission, et pour saisir une deuxième grandeur de perte sur la base du signal d'émission et d'un signal de réception lors de la sélection de la deuxième configuration de saisie qui émet un signal d'émission, le processeur étant configuré pour sélectionner la configuration de saisie présentant la grandeur de perte la plus basse pour saisir le paramètre d'imagerie du sang.

2.  Dispositif de saisie selon la revendication 1, dans lequel, l'émetteur (101) est configuré pour émettre le signal d'émission au moyen de l'antenne d'émission de la première configuration de saisie dans le cas de la sélection de la première configuration de saisie et dans lequel le récepteur (105) est configuré pour recevoir le signal de réception au moyen de l'antenne de réception de la première configuration de saisie lors de la sélection de la première configuration de saisie, dans lequel l'émetteur (101) est configuré pour émettre le signal d'émission au moyen de l'antenne d'émission de la deuxième configuration de saisie dans le cas de la sélection de la deuxième configuration de saisie et dans lequel le récepteur (105) est configuré pour recevoir le signal de réception au moyen de l'antenne de réception de la première configuration de saisie lors de la sélection de la deuxième configuration de saisie, et dans lequel le détecteur de perte (107) est configuré pour saisir la première grandeur de perte sur la base du signal d'émission et du signal de réception de la première configuration de saisie et pour saisir la deuxième grandeur de perte sur la base du signal d'émission et du signal de réception de la deuxième configuration de saisie.

3.  Dispositif de saisie selon l'une des revendications précédentes, dans lequel le processeur (109) est configuré pour comparer la première grandeur de perte à la deuxième grandeur de perte pour déterminer quelle est la plus petite des deux grandeurs de perte.

4.  Dispositif de saisie selon l'une des revendications précédentes, qui comporte en outre une matrice de commutation (211), en particulier une matrice de commutation (211) apte à être commandée par le processeur (109) et configurée pour relier une sortie d'une des antennes de réception au détecteur de perte (107).

5.  Dispositif de saisie selon l'une des revendications précédentes, dans lequel l'émetteur (101) comporte un générateur (202) de signaux d'émission et dans lequel une sortie du générateur (202) de signaux d'émission peut être reliée à une des antennes d'émission par une matrice de commutation (209), notamment par une matrice de commutation (209) apte à être commandée par le processeur (109).

6.  Dispositif de saisie selon l'une des revendications précédentes, dans lequel l'émetteur (101) est configuré pour, lors de l'utilisation de la première configuration de saisie et lors de l'utilisation de la deuxième configuration de saisie, émettre des signaux d'émission de même fréquence, en particulier à une fréquence comprise dans une plage de fréquence située entre 1 GHz et 15 GHz, ou en même mode, en particulier en mode transversal-électrique ou en mode transversal-magnétique.

7.  Dispositif de saisie selon l'une des revendications précédentes, dans lequel l'émetteur (101) comporte au moins deux antennes d'émission (203) ou dans lequel le récepteur comporte au moins deux antennes de réception (207), en particulier des antennes dipolaires, des antennes en cadre ou des antennes dites "en patch".

8.  Dispositif de saisie selon l'une des revendications précédentes, dans lequel le détecteur de perte (107) est un analyseur de réseau, en particulier un analyseur vectoriel ou scalaire de réseau, ou un détecteur de puissance.

9.  Dispositif de saisie selon l'une des revendications précédentes, dans lequel pour la saisie du paramètre d'imagerie du sang, l'émetteur (101) est configuré pour injecter un premier signal d'émission à une première fréquence et un deuxième signal d'émission à une deuxième fréquence dans le vaisseau sanguin en recourant à l'antenne d'émission de la configuration de saisie sélectionnée, dans lequel le récepteur (105) est configuré pour recevoir un premier signal de réception à la première fréquence et un deuxième signal de réception à la deuxième fréquence en recourant à l'antenne de réception de la configuration de saisie sélectionnée, dans lequel le détecteur de perte (107) est configuré pour déterminer une première grandeur de perte sur la base du premier signal d'émission et du premier signal de réception à la première fréquence et pour déterminer une deuxième grandeur de perte sur la base du deuxième signal d'émission et du deuxième signal de réception à la deuxième fréquence; et dans lequel le processeur (109) est configuré pour déterminer un premier décalage de fréquence de la première grandeur de perte par rapport à une première grandeur de perte de référence, pour déterminer un deuxième décalage de fréquence de la deuxième grandeur de perte par rapport à une deuxième grandeur de perte de référence et pour déterminer le paramètre d'imagerie du sang sur la base du premier décalage en fréquence et du deuxième décalage en fréquence.

10. Dispositif de saisie selon l'une des revendications précédentes, dans lequel pour la saisie du paramètre d'imagerie du sang, l'émetteur (101) est configuré pour injecter un premier signal d'émission à une première fréquence et un deuxième signal d'émission à une deuxième fréquence dans le vaisseau sanguin en recourant à l'antenne d'émission de la configuration de saisie sélectionnée; dans lequel le récepteur (105) est configuré pour recevoir un premier signal de réception à la première fréquence et un deuxième signal de réception à la deuxième fréquence en recourant à l'antenne de réception de la configuration de saisie sélectionnée; et dans lequel le processeur (109) est configuré pour déterminer une première grandeur de perte sur la base du première signal d'émission et du premier signal de réception, pour déterminer une deuxième grandeur de perte sur la base du deuxième signal d'émission et du deuxième signal de réception, pour déterminer une constante de temps de relaxation d'un composant du sang en fonction de la fréquence qui présente la plus grande grandeur de perte et pour déterminer le paramètre d'imagerie du sans sur la base d'une dépendance préalablement connue de la constante de temps de relaxation vis-à-vis du paramètre d'imagerie du sang.

11. Dispositif de saisie selon l'une des revendications précédentes, dans lequel le détecteur de perte (107) est configuré pour exécuter une mesure à deux portes pour déterminer la première grandeur de perte et la deuxième grandeur de perte, en particulier au moyen de la mesure à deux portes pour déterminer un facteur $S_{21}$ de transmission avant.

12. Dispositif de saisie selon l'une des revendications précédentes, dans lequel le détecteur de perte (107) est configuré pour déterminer la première grandeur de perte et la deuxième grandeur de perte sur la base de la formule ci-dessous:

$$P_{Verlust} = 1 - \left| S_{11} \right|^2 - \left| S_{21} \right|^2,$$

dans laquelle $P_{Verlust}$ désigne la grandeur de perte particulière et $S_{11}$ le facteur de réflexion d'entrée et $S_{21}$ le facteur de transmission avant.

13. Dispositif de saisie selon l'une des revendications précédentes, dans lequel l'émetteur (101) est configuré pour injecter le signal d'émission sous la forme d'un mode ou d'un type d'onde, en particulier sous la forme d'une onde transversale-électrique (TE), d'une onde transversale-magnétique (TM), ou d'une onde transversale-électromagnétique (TEM) ou d'une onde HE, en particulier tangentiellement ou transversalement par rapport à une extension du vaisseau sanguin ou par rapport à une direction d'écoulement du sang.

14. Dispositif de saisie selon l'une des revendications précédentes, dans lequel le paramètre d'imagerie du sang est la concentration d'un composant du sang, en particulier un sucre, par exemple le glucose, ou de l'oxygène.

15. Procédé de saisie d'un paramètre d'imagerie du sang dans un vaisseau sanguin par recours à un émetteur qui présente plusieurs antennes d'émission qui émettent au moins un signal d'émission et d'un récepteur qui présente plusieurs antennes de réception qui reçoivent au moins un signal de réception, le procédé comportant:

sélectionner une première configuration de saisie qui comprend une des antennes d'émission et une des antennes de réception;
dans le cas de la sélection de la première configuration de saisie pour émettre un signal d'émission, saisir une première grandeur de perte sur la base du signal d'émission et d'un signal de réception,
sélectionner une deuxième configuration de saisie qui comporte une des antennes d'émission et une des antennes de réception;
dans le cas de la sélection de la deuxième configuration de saisie pour émettre un signal d'émission, saisir une deuxième grandeur de perte sur la base du signal d'émission et d'un signal de réception; et

sélectionner la configuration de saisie qui présente la grandeur de perte le plus petit pour saisir le paramètre d'imagerie du sang.

103

Sender

Empfänger

101

105

Verlustdetektor

107

Speicher

109

Prozessor

111

100

Fig. 1

Fig. 2

Fig. 3A　　　　Fig. 3B　　　　Fig. 3C

Fig. 4A

Fig. 4B

413   415

411   407

Blutgefäß

$E_{longitudinal}$

417

Fig. 4C

407

419

Blutgefäß

$H_{longitudinal}$

420

Fig. 4D

507

508

501

503

505

Fig. 5A

509      513

511

515

Fig. 5B

Fig. 6A

Fig. 6B

703

701

705

Fig. 7A

707

709

705

H

705

711

703

703

Fig. 7B

Fig. 8

Fig. 9

Fig. 10

Fig. 11

1203   1205

1201   1206

1207

1209

Fig. 12

Fig. 13

Fig. 14

| Durchm. El | A | B | C | D | E | F | G |
|---|---|---|---|---|---|---|---|
| 6 mm | <1 | +2 | -1 | -9 | -17 | +11 | <1 |
| 3,4 mm | -1 | +4 | -1 | -13 | - | - | -10 |

Fig. 15

Fig. 16

Fig. 17

Fig. 18

Fig. 19

Fig. 20

Fig. 21

Fig. 22

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 20077003955 A1
- US 6332087 B1
- WO 20101105373 A1
- US 201010069731 A1
- US 20080200790 A1

**In der Beschreibung aufgeführte Nicht-Patentliteratur**

- **ANDREAS CADUFF et al.** Non-invasive glucose monitoring in patients with Type 1 diabetes: A multi-sensor system combining sensors for dielectric and optical characterization of skin. *Biosensors and Bioelectronics,* 2009, vol. 24, 2778-2784
- **BUFORD RANDAL JEAN et al.** A microwave frequency sensor for non-invasive blood-glucose measurement. *SAS 2008 - IEEE Sensors Applications Symposium, Atlanta, GA,* 12. Februar 2008
- **M. MCCLUNG.** Calibration methodology for a microwave non-invasive glucose sensor. *Master Thesis,* Mai 2008
- **NETTER, F. N.** Atlas der Anatomie. Thieme Verlag, 2006